# EUROPEAN PATENT SPECIFICATION

(11) **EP 1 797 953 B1**
(45) Date of publication and mention of the grant of the patent: **05.01.2011**
(21) Application number: 05766519.2
(22) Date of filing: 15.07.2005
(51) Int. Cl.: B01J 31/26, C07C 27/00, C07C 29/48, C07C 33/03, C07C 45/28, C07C 47/21, C07C 51/285, C07C 61/04, C07C 61/06, C07C 62/24, C07C 67/31, C07C 67/313, C07C 69/145, C07C 69/747, C07B 61/00

(54) **PROCESS FOR PRODUCING OXYGENIC COMPOUND**
VERFAHREN ZUR HERSTELLUNG EINER SAUERSTOFFHALTIGEN VERBINDUNG
PROCEDE POUR LA PREPARATION DE 4-AZASTEROIDES

(30) Priority: 20.07.2004 JP 2004211133
(43) Date of publication of application: 20.06.2007
(73) Proprietor: Sumitomo Chemical Company, Limited, Tokyo 104-8260 (JP)
(72) Inventor: HAGIYA, Koji, 5670833 (JP)
(74) Representative: Henkel, Feiler & Hänzel
(86) International application number: PCT/JP2005/013526
(87) International publication number: WO 2006/009266

(56) References cited:
- EP-A- 1 609 777
- DE-A1- 1 620 159
- JP-A- 2003 231 677
- US-A- 4 182 901
- MLOCHOWSKI ET AL: POLISH J. CHEM., vol. 76, 2002, pages 537-542, XP002503303

## Description

### Technical Field

The present invention relates to a method for producing an oxygen-containing compound.

### Background Art

An oxygen-containing compound such as an α-hydroxyolefin compound and an α-oxoolefin compound obtained by oxidizing the carbon atom at α-position of an olefin compound having two or more hydrogen atoms on the carbon atom at α-position of a carbon-carbon double bond is a very important compound as various chemicals and synthetic intermediate thereof. For example, 3,3-dimethyl-2-E-(2-formyl-1-propenyl)cyclopropanecarboxylates and 3,3-dimethyl-2-E-(2-carboxy-1-propenyl)cyclopropanecarboxylates obtained by oxidizing a carbon atom at α-position of the 2-methyl-1-propenyl group at 2-position of 3,3-dimethyl-2-(2-methyl-1-propenyl)cyclopropanecarboxylates are important chrysanthemic acid derivatives as an acid part of household agents for epidemic prevention and insecticides which is known as pyrethrates as described in Proc. Japan Acad., 32, 353 (1956) and Synthetic Pyrethroid Insecticides: Structure and Properties,3(1990). E,E-2,6-dimethyl-8-acetoxy-2,6-octadien-1-ol obtained by oxidizing a terminal methyl group of geranyl acetate is useful as an intermediate in natural-product synthesis as described in Tetrahedron Letters, 42, 2205 (2001).

A cycloalkanecarboxylic acid compound obtained by oxidizing a cycloalkanone compound is also important as a synthetic intermediate of natural products and pharmaceuticals as described in Synth. Commun., 29, 2281 (1999).

Proc. Japan Acad., 32, 353 (1956) discloses a method for producing 3,3-dimethyl-2-(2-E-formyl-1-propenyl)cyclopropanecarboxylate comprising reacting 3,3-dimethyl-2-(2-methyl-1-propenyl)cyclopropanecarboxylate with selenium dioxide.

J. Amer. Chem. Soc., 99, 5526 (1977) and Tetrahedron Letters, 42, 2205 (2001) disclose a method comprising reacting an olefin compound having two or more hydrogen atoms on a carbon atom at α-position of a carbon-carbon double bond such as geranyl acetate with an organic hydroperoxide in the presence of selenium oxide catalyst. WO 04/85376 discloses a method comprising reacting the above-mentioned olefin compound with an organic hydroperoxide in the presence of a selenium compound catalyst in an ionic liquid.

Synth. Commun., 29, 2281 (1999) and J. Org. Chem., 22, 1680 (1957) disclose a method for producing a cyclopropanecarboxylic acid compound comprising reacting a cycloalkanone compound with hydrogen peroxide in the presence of selenium dioxide catalyst.

### Disclosure of the Invention

The present invention provides an oxidation catalyst composition comprising a mixture of a selenium compound, a nitrogen-containing aromatic compound and an acid as further defined in the claims, and a method for producing an oxygen-containing compound comprising reacting an organic compound with an oxidizing agent in the presence of the oxidation catalyst composition as further defined in the claims.

### Best Mode for Carrying Out the Present Invention

First, the oxidation catalyst composition comprising the mixture of the selenium compound, the nitrogen-containing aromatic compound and the acid as further defined in the claims will be illustrated.

The selenium compound is selenium dioxide or an alkali metal selenite. Examples of the alkali metal selenite include sodium selenite and potassium selenite.

A commercially available selenium compound can be used as it is. The selenium compound may be used alone and two or more kinds thereof may be used.

Thenitrogen-containing aromatic compound is pyridine, 2-methylpyridine, 3-butylpyridine, collidine, quinoline, 1,10-phenanthroline, imidazole, Nor methylimidazole, 2-ethyl-4-methylimidazole or benzimidazole.

The nitrogen-containing aromatic compounds may be used alone, and two or more thereof may be used. The amount thereof to be used is usually 0.5 to 10 moles, preferably 0.9 to 8 moles relative to 1 mole of the selenium compound.

The acid is phosphoric acid, tetrafluoroboric acid or benzenesulfonic acid. As the acid, a commercially available one may be used as it is or after diluting with water or an organic solvent. The acid whose form is hydrate may be used. The acids may be used alone and two or more thereof may be used.

The amount of the acid to be used is usually 0.5 to 2 moles relative to 1 mole of the nitrogen-containing aromatic compound.

The mixture of the selenium compound, the nitrogen-containing aromatic compound and the acid can be usually obtained by mixing the selenium compound, the nitrogen-containing aromatic compound and the acid. The mixing order is not particularly limited. The temperature of the mixing is usually 0 to 200°C.

The oxidation catalyst composition of the present invention may include the mixture of the selenium compound, the nitrogen-containing aromatic compound and the acid, and if necessary, it may include the other component such as a solvent. Examples of the solvent include an aliphatic hydrocarbon solvent such as hexane and heptane; an aromatic hydrocarbon solvent such as toluene and xylene; an ether solvent such as diethyl ether, methyl tert-butyl ether and tetrahydrofuran; an ester solvent such as ethyl acetate; an alcohol solvent such as tert-butanol; a halogenated hydrocarbon solvent such as chloroform, dichloromethane and chlorobenzene; a nitrile solvent such as acetonitrile and propionitrile; an amide solvent such as N,N-dimethylformamide and N,N-dimethylacetamide; a sulfur-containing solvent such as dimethylsulfoxide, dimethylsulfone and sulfolane; water; and an ionic liquid. These solvents may be used alone or in the form of a mixture. Water, the ionic liquid and a mixture of water and the ionic liquid are preferable, and water and the mixture of water and the ionic liquid are more preferable. When selenium dioxide is used as the selenium compound, it is preferred to be used water or a mixed solvent of water and the above-mentioned solvent. The amount of the solvent to be used is not particularly limited.

In the present invention, the ionic liquid means a salt which consists of an organic cation and an anion, which has a melting point of 100°C or less and which is stable to hold liquid state without decomposing until 300°C.

Examples of the organic cation include a substituted imidazolium cation, an alkyl-substituted pyridinium cation, a quarternary ammonium cation, a quarternary phosphonium cation and a tertiary sulfonium cation. The substituted imidazolium cation and the alkyl-substituted pyridinium cation are preferable.

The substituted imidazolium cation means an imidazoliumu cation of which at least one nitrogen atom on the imidazoline ring is bonded to a C1-C8 alkyl group or groups, a C1-C8 alkyl group or groups substituted with a C1-C8 alkoxy group, a C1-C8 haloalkyl group or groups, or a C1-C8 alkyl group or groups substituted with a C2-C7 alkoxycarbonyl group. Examples of the C1-C8 alkyl group include a methyl, ethyl, propyl, isopropyl, butyl, isobutyl and pentyl group. Examples of the C1-C8 alkyl group substituted with the C1-C8 alkoxy group include a methoxymethyl, ethoxymethyl and methoxyethyl group. Examples of the C1-C8 haloalkyl group include a chloromethyl, fluoromethyl and trifluoromethyl group. Examples of the C1-C8 alkyl group substituted with the C2-C7 alkoxycarbonyl group include a methoxycarbonylmethyl group.

Examples of the substituted imidazolium cation include a 1-methyl-3-methylimidazolium, 1-methyl-3-ethylimidazolium, 1-methyl-3-butylimidazolium, 1-methyl-3-isobutylimidazolium, 1-methyl-3-(methoxyethyl)imidazolium, 1-ethyl-3-ethylimidazolium, 1-ethyl-3-butylimidzolium, 1-ethyl-2,3-dimethylimidazolium, 1-ethyl-3,5-dimethylimidazolium, 1,3-diethyl-5-methylimidazolium and 1-ethylimidazolium cation.

The alkyl-substituted pyridinium cation means a pyridinium cation of which at least nitrogen atom on the pyridine ring is bonded to a C1-C8 alkyl group or groups, a C1-C8 alkyl group or groups substituted with a C1-C8 alkoxy group, a C1-C8 haloalkyl group or groups, or a C1-C8 alkyl group or groups substituted with a C2-C7 alkoxycarbonyl group. Examples of the alkyl-substituted pyridinium cation include an N-methylpyridinium, N-ethylpyridinium, N-propylpyridinium, N-butylpyridinium, N-butyl-4-methylpyridinium, N-isobutylpyridinium and N-pentylpyridinium cation.

The quarternary ammonium cation means an ammonium cation wherein same or different four groups selected from a C1-C8 alkyl group or groups, a C1-C8 alkyl group or groups substituted with a C1-C8 alkoxy group, a C1-C8 haloalkyl group or groups, and a C1-C8 alkyl group or groups substituted with a C2-C7 alkoxycarbonyl group are bonded to a nitrogen atom. Examples of the quarternary ammonium cation include a trimethylpentylammonium, trimethylhexylammonium, trimethylheptylammonium, trimethyloctylammonium and triethylpentylammonium cation.

The quarternary phosphonium cation means a phosphonium cation wherein same or different four groups selected from a C1-C8 alkyl group or groups, a C1-C8 alkyl group or groups substituted with a C1-C8 alkoxy group, a C1-C8 haloalkyl group or groups, and a C1-C8 alkyl group or groups substituted with a C2-C7 alkoxycarbonyl group are bonded to a phosphorous atom. Examples of the quarternary phosphonium cation include a trimethylpentylphosphonium and tetrabutylphosphonium cation.

The tertiary sulfonium cation means a sulfonium cation wherein same or different three groups selected from a C1-C8 alkyl group or groups, a C1-C8 alkyl group or groups substituted with a C1-C8 alkoxy group, a C1-C8 haloalkyl group or groups, and a C1-C8 alkyl group or groups substituted with a C2-C7 alkoxycarbonyl group are bonded to a sulfur atom. Examples of the tertiary sulfonium cation include a triethylsulfonium, tributylsulfonium and tripropylsulfonium cation.

Examples of the anion include a tetrafluoroborate anion, a halogen anion, a hexafluorophosphate anion, a bis(perfluoroalkanesulfonyl)amide anion, an alkylcarboxylate anion and an alkanesulfonate anion.

Examples of the ionic liquid include 1-methyl-3-methylimidazolium tetrafluoroborate, 1-methyl-3-ethylimidazolium tetrafluoroborate, 1-methyl-3-butylimidazolium tetrafluoroborate, 1-methyl-3-isobutylimidazolium tetrafluoroborate, 1-methyl-3-(methoxyethyl)imidazolium tetrafluoroborate, 1-ethyl-3-ethylimidazolium tetrafluoroborate, 1-ethyl-3-butylimidzolium tetrafluoroborate, 1-ethyl-2,3-dimethylimidazolium tetrafluoroborate, 1-ethyl-3,5-dimethylimidazolium tetrafluoroborate, 1,3-diethyl-5-methylimidazolium tetrafluoroborate, 1-ethylimidazolium tetrafluoroborate, N-methylpyridinium tetrafluoroborate, N-ethylpyridinium tetrafluoroborate, N-propylpyridinium tetrafluoroborate, N-butylpyridinium tetrafluoroborate, N-butyl-4-methylpyridinium tetrafluoroborate, N-isobutylpyridinium tetrafluoroborate, N-pentylpyridinium tetrafluoroborate, trimethylpentylammonium tetrafluoroborate, trimethylhexylammonium tetrafluoroborate, trimethylheptylammonium tetrafluoroborate, trimethyloctylammonium tetrafluoroborate, triethylpentylammonium tetrafluoroborate, trimethylpentylphosphonium tetrafluoroborate, tetrabutylphosphonium tetrafluoroborate, triethylsulfonium tetrafluoroborate, tributylsulfonium tetrafluoroborate and tripropylsulfonium tetrafluoroborate, and those wherein the tetrafluoroborate anion of the above-mentioned ionic liquid is replaced with a chloride anion, a bromide anion, an iodide anion, a hexafluorophosphate anion, a bis(perfluoroalkanesulfonyl)amide anion, an alkylcarboxylate anion or an alkanesulfonate anion such as 1-methyl-3-methylimidazolium chloride, 1-methyl-3-methylimidazolium bromide, 1-methyl-3-methylimidazolium iodide, 1-methyl-3-methylimidazolium hexafluorophosphate, 1-methyl-3-methylimidazolium bis(perfluoroalkanesulfonyl)amide, 1-methyl-3-methylimidazolium alkylcarboxylate and 1-methyl-3-methylimidazolium alkanesulfonate.

A commercially available ionic liquid may be used and one produced according to a method, for example, described in Tetrahedron, 59, 2253 (2003) may be used.

The containing oxidation catalyst composition of the present invention has a catalytic activity on the oxidation reaction of an organic compound and an oxygen-containing compound to which the organic compound is oxidized can be produced by reaction of the organic compound and an oxidizing agent in the presence of the oxidation catalyst composition.

Next, a method for producing the oxygen-containing compound comprising reacting the organic compound with the oxidizing agent in the presence of the oxidation catalyst composition.

The organic compound is an olefin compound having two or more hydrogen atoms on the carbon atom at α-position of a carbon-carbon double bond (hereinafter, simply referred to as the olefin compound) or a cycloalkanone compound.

The oxidizing agent is an organic hydroperoxide compound such as tert-butyl hydroperoxide, tert-amyl hydroperoxide, cumene hydroperoxide and cymene hydroperoxide or hydrogen peroxide. These are accordingly selected depending on the kind of the organic compound.

In the case that the organic compound is the olefin compound, at least one oxygen-containing compound selected from an α-hydroxyolefin compound and an α-oxoolefin is formed by using the organic hydroperoxide as the oxidizing agent. In the case that the organic compound is the cycloalkanone compound, a cycloalkanecarboxylic acid compound is obtained by using hydrogen peroxide as the oxidizing agent.

The reaction of the olefin compound and the organic hydroperoxide will be illustrated below.

The olefin compound may be an olefin compound having a carbon-carbon double bond within a molecule and having two or more hydrogen atoms on the carbon atom at α-position thereof. Examples thereof include an olefin compound represented by the formula (1) (hereinafter, simply referred to as the olefin compound (1)): 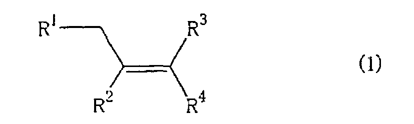 wherein R¹ represents a halogen atom: a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group which is optionally substituted with a halogen atom or atoms, a C1-C20 alkoxy group or groups, or a C6-C10 aryloxy group or groups; or a C7-C12 aralkyl group which is optionally substituted with a halogen atom or atoms, a C1-C20 alkoxy group or groups, or a C6-C10 aryloxy group or groups, and herein, the above-mentioned C1-C20 alkoxy group, C6-C10 aryloxy group, C7-C12 aralkyloxy group, C2-C10 alkoxycarbonyl group and C7-C12 aryloxycarbonyl group may be substituted with a halogen atom or atoms, a C1-C20 alkoxy group or groups, or a C6-C10 aryloxy group or groups, R², R³ and R⁴ are the same or different and each independently represent a hydrogen atom; a halogen atom; a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C1-C20 alkoxy group; a C2-C12 alkenyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group; a C6-C10 aryloxy group; a C7-C12 aralkyl group; a C7-C12 aralkyloxy group; a C2-C10 acyl group; a C2-C10 alkoxycarbonyl group; a C7-C12 aryloxycarbonyl group; a C8-C12 aralkyloxycarbonyl group; or a carboxyl group, and herein, the above-mentioned C1-C20 alkoxy group, C2-C10 acyl group, C6-C10 aryl group, C6-C10 aryloxy group, C7-C12 aralkyl group, C7-C12 aralkyloxy group, C2-C10 alkoxycarbonyl group, C7-C12 aryloxycarbonyl group and C8-C12 aralkyloxycarbonyl group may be substituted with a halogen atom or atoms, a C1-C20 alkoxy group or groups, or a C6-C10 aryloxy group or groups, and at least one pair selected from R¹ and R³, R² and R⁴, R¹ and R² and R³ and R⁴ may be bonded to form a ring, an olefin compound represented by the formula (4) (hereinafter, simply referred to as the olefin compound (4)): 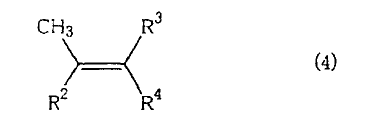 wherein R², R³ and R⁴ respectively represent the same as described above, and herein, R² and R⁴ or R³ and R⁴ may be bonded to form a ring, and the like. Specific examples of the olefin compound (4) include a chrysanthemic acid compound represented by the formula (8)(hereinafter, simply referred to as the chrysanthemic acid compound (8)): 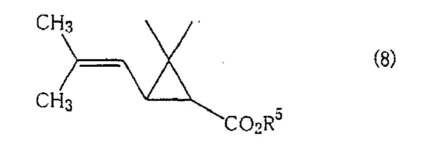 wherein R⁵ represents a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group; a C7-C12 aralkyl group; or a hydrogen atom, and herein, the above-mentioned C1-C20 alkoxy group, C6-C10 aryloxy group, C7-C12 aralkyloxy group, C2-C10 alkoxycarbonyl group, C7-C12 aryloxycarbonyl group and C7-C12 aralkyl group may be substituted with a halogen atom or atoms, a C1-C20 alkoxy group or groups, or a C6-C10 aryloxy group or groups.

In the case that the olefin compound (1) is used as the olefin compound, an alcohol compound represented by the formula (2) (hereinafter, simply referred to as the alcohol compound (2)): 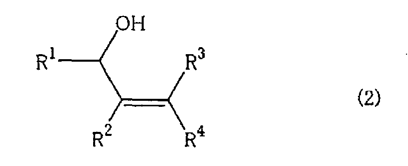 wherein R¹, R², R³ and R⁴ are the same as the described above, is produced as the α-hydroxyolefin compound, and a ketone compound represented by the formula (3) (hereinafter, simply referred to as the ketone compound (3)): 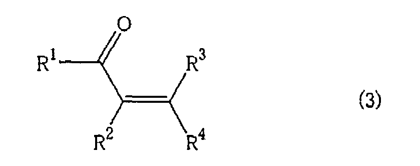 wherein R¹, R², R³ and R⁴ are the same as the describes above, is produced as the α-oxoolefin compound.

In the case that the olefin compound is the olefin compound (4), an alcohol compound represented by the formula (5) (hereinafter, simply referred to as the alcohol compound (5)): 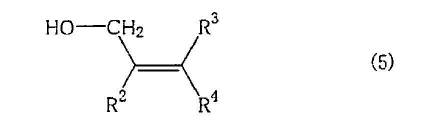 wherein R², R³ and R⁴ are the same as the described above, is produced as the α-hydroxyolefin compound, and at least one selected from an aldehyde compound represented by the formula (6) (hereinafter, simply referred to as the aldehyde compound (6)): 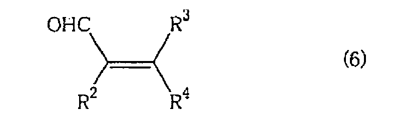 wherein R², R³ and R⁴ are the same as the described above, and a carboxylic acid compound represented by the formula (7) (hereinafter, simply referred to as the carboxylic acid compound (7)): 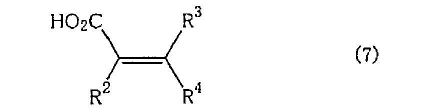 wherein R², R³ and R⁴ are the same as the described above, is produced as the α-oxoolefin compound.

In the case that the chrysanthemic acid compound (8) is used as the olefin compound (4), an alcohol compound represented by the formula (9) (hereinafter, simply referred to as the alcohol compound (9)): 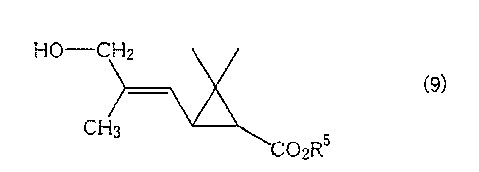 wherein R⁵ is the same as the described above, is produced as the α-hydroxyolefin compound, and at least one selected from an aldehyde compound represented by the formula (10) (hereinafter, simply referred to as the aldehyde compound (10))_{:} 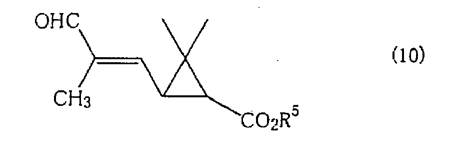 wherein R⁵ is the same as the described above, and a carboxylic acid compound represented by the formula (11) (hereinafter, simply referred to as the carboxylic acid compound (11)): 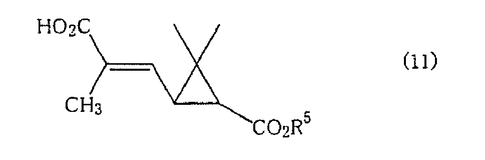 wherein R⁵ is the same as the described above, is produced as the α-oxoolefin compound.

Examples of the halogen atom include a fluorine, chlorine, and bromine atom.

Examples of the C1-C20 alkoxy group include a methoxy, ethoxy, propoxy, isopropoxy, butoxy, isobutoxy, sec-butoxy, tert-butoxy, pentyloxy, hexyloxy, n-decyloxy and cyclopentyloxy group. The C1-C20 alkoxy group may be substituted with a halogen atom or atoms, a C1-C20 alkoxy group or groups, or a C6-C10 aryloxy group or groups. Examples of the C1-C20 alkoxy group substituted with the halogen atom or atoms, the C1-C20 alkoxy group or groups, or the C6-C10 aryloxy group or groups include a chloromethoxy, fluoromethoxy, trifluoromethoxy, methoxymethoxy, ethoxymethoxy, methoxyethoxy and phenoxymethoxy group.

Examples of the C2-C10 acyl group include an acetyl, propionyl, benzoyl and benzylcarbonyl group. The C2-C10 acyl group may be substituted with the halogen atom or atoms, the C1-C20 alkoxy group or groups, or the C6-C10 aryloxy group or groups.

Examples of the C6-C10 aryloxy group include a phenoxy, 2-methylphenoxy, 4-methylphenoxy and naphthoxy group. The C6-C10 aryloxy group may be substituted with the halogen atom or atoms, the C1-C20 alkoxy group or groups, or the C6-C10 aryloxy group or groups. Examples of the C6-C10 aryloxy group substituted with the halogen atom or atoms, the C1-C20 alkoxy group or groups, or the C6-C10 aryloxy group or groups include a 4-chlorophenoxy, 4-methoxyphenoxy and 3-phenoxyphenoxy group.

Examples of the C7-C12 aralkyloxy group include a benzyloxy and 4-methylbenzyloxy group. The C7-C12 aralkyloxy group may be substituted with the halogen atom or atoms, the C1-C20 alkoxy group or groups, or the C6-C10 aryloxy group or groups. Examples of the C7-C12 aralkyloxy group substituted with the halogen atom or atoms, the C1-C20 alkoxy group or groups, or the C6-C10 aryloxy group or groups include a 4-chlorobenzyloxy, 4-methoxybenzyloxy, 3-phenoxybenzyloxy, 2,3,5,6-tetrafluorobenzyloxy, 2,3,5,6-tetrafluoro-4-methylbenzyloxy, 2,3,5,6-tetrafluoro-4-methoxybenzyloxy and 2,3,5,6-tetrafluoro-4-methoxymethylbenxyloxy group.

Examples of the C2-C10 alkoxycarbonyl group include a methoxycarbonyl, ethoxycarbonyl and isopropoxycarbonyl group. Examples of the C7-C12 aryloxycarbonyl group include a phenoxycarbonyl group. The C2-C10 alkoxycarbonyl group and the C7-C12 aryloxycarbonyl group may be substituted with the halogen atom or atoms, the C1-C20 alkoxy group or groups, or the C6-C10 aryloxy group or groups.

Examples of the C1-C20 alkyl group which is optionally substituted with the halogen atom or atoms, the C1-C20 alkoxy group or groups, the C2-C10 acyl group or groups, the C6-C10 aryloxy group or groups, the C7-C12 aralkyloxy group or groups, the C2-C10 alkoxycarbonyl group or groups, the C7-C12 aryloxycarbonyl group or groups, or the carboxyl group or groups include a methyl, ethyl, propyl, isopropyl, butyl, isobutyl, sec-butyl, tert-butyl, pentyl, decyl, cyclopropyl, 2,2-dimethylcyclopropyl, cyclopentyl, cyclohexyl, menthyl, chloromethyl, fluoromethyl, trifluoromethyl, methoxymethyl, ethoxymethyl, methoxyethyl and methoxycarbonylmethyl group.

Examples of the C6-C10 aryl group which is optionally substituted with the halogen atom or atoms, the C1-C20 alkoxy group or groups, or the C6-C10 aryloxy group or groups include a phenyl, 2-methylphenyl, 4-methylphenyl, 4-chlorophenyl, 4-methoxyphenyl and 3-phenoxyphenyl group.

Examples of the C7-C12 aralkyl group which is optionally substituted with the halogen atom or atoms, the C1-C20 alkoxy group or groups, or the C6-C10 aryloxy group or groups include a benzyl, 4-chlorobenzyl, 4-methylbenzyl, 4-methoxybenzyl, 3-phenoxybenzyl, 2,3,5,6-tetrafluorobenzyl, 2,3,5,6-tetrafluoro-4-methylbenzyl, 2,3,5,6-tetrafluoro-4-methoxybenzyl and 2,3,5,6-tetrafluoro-4-methoxymethylbenzyl group.

Examples of the C2-C12 alkenyl group which is optionally substituted with the halogen atom or atoms, the C1-C20 alkoxy group or groups, the C2-C10 acyl group or groups, the C6-C10 aryloxy group or groups, the C7-C12 aralkyloxy group or groups, the C2-C10 alkoxycarbonyl group or groups, the C7-C12 aryloxycarbonyl group or groups, or the carboxyl group or groups include an ethenyl, 1-propenyl, 1-methylethenyl, 1-butenyl, 1-methyl-1-propenyl, 2-methyl-1-propenyl, 1-pentenyl, 1-hexenyl, 1-decenyl, 2-cyclopentenyl, 2-cyclohexenyl, 3-acetoxy-1-methyl-1-propenyl, 2,2-dichloroethenyl, 3-bromo-1-methyl-1-propenyl, 5-oxo-1-methyl-1-hexenyl and 3-methoxy-1-methyl-1-propenyl group.

Examples of the olefin compound (1) include 1-hexene, 1-heptene, 1-octene, 1-dodecene, cyclopentene, cyclohexene, cycloheptene, cyclooctene, 3-methylcyclopentene, 4-methylcyclopentene, 3,4-dimethylcyclopentene, 3-chlorocyclopentene, 3-methylcyclohexene, 1,7-octadiene, 1,2,3,4-tetrahydrophthalic anhydride, indene, methylenecyclobutane, methylenecyclopentane, β-pinene, α-methylene-γ-butyrolactone and cyclohexylidenecyclohexane.

Examples of the olefin compound (4) include geranyl acetate, geranyl benzoate, geranyl methyl ether, geranyl benzyl ether, geranyl phenyl sulfone, 2-hexene, α-methylstyrene, pulegone, isophorone, 2-carene, 3-carene, α-pinene and the following chrysanthemic acid compound (8).

Examples of the chrysanthemic acid compound (8) include 3,3-dimethyl-2-(2-methyl-1-propenyl)cyclopropancarboxylic acid, methyl 3,3-dimethyl-2-(2-methyl-1-propenyl)cyclopropancarboxylate, ethyl 3,3-dimethyl-2-(2-methyl-1-propenyl)cyclopropancarboxylate, isopropyl 3,3-dimethyl-2-(2-methyl-1-propenyl)cyclopropancarboxylate, tert-butyl 3,3-dimethyl-2-(2-methyl-1-propenyl)cyclopropancarboxylate, cyclohexyl 3,3-dimethyl-2-(2-methyl-1-propenyl)cyclopropancarboxylate, menthyl 3,3-dimethyl-2-(2-methyl-1-propenyl)cyclopropancarboxylate, benzyl 3,3-dimethyl-2-(2-methyl-1-propenyl)cyclopropancarboxylate, 4-chlorobenzyl 3,3-dimethyl-2-(2-methyl-1-propenyl)cyclopropancarboxylate, 2,3,5,6-tetrafluorobenzyl 3,3-dimethyl-2-(2-methyl-1-propenyl)cyclopropancarboxylate, 2,3,5,6-tetrafluoro-4-methylbenzyl 3,3-dimethyl-2-(2-methyl-1-propenyl)cyclopropancarboxylate, 2,3,5,6-tetrafluoro-4-methoxybenzyl 3,3-dimethyl-2-(2-methyl-1-propenyl)cyclopropancarboxylate, 2,3,5,6-tetrafluoro-4-methoxymethylbenzyl 3,3-dimethyl-2-(2-methyl-1-propenyl)cyclopropancarboxylate and 3-phenoxybenzyl 3,3-dimethyl-2-(2-methyl-1-propenyl)cyclopropancarboxylate.

A commercially available olefin compound may be used and one produced accordingly using a known method such as the Wittig reaction may be used.

Among them, there are olefin compounds having an optical isomer and an optical isomer can be used alone and a mixture thereof.

The chrysanthemic acid compound (8) has a compound having the -CO₂R⁵ group and the 2-methyl-1-propenyl group on the same side with respect to the cyclopropane ring plane (hereinafter, referred as the cis-isomer) and the compound having the -CO₂R⁵ group and the 2-methyl-1-propenyl group on the opposite side (hereinafter, referred as the trans-isomer). In the present invention, any one of them may be used and a mixture thereof may be used. When the mixture thereof is used, a mixed ratio of the cis-isomer and the trans-isomer is not particularly limited.

For example, when cyclohexane is used as the olefin compound (1), at least one oxygen-containing compound selected from 2-cyclohexenol and 2-cyclohexenone. When isophorone is used as the olefin compound (4), at least one oxygen-containing compound selected from 3-hydroxymethyl-5,5-dimethyl-2-cyclohexen-1-one, formylisophorone and 5,5-dimethyl-3-oxo-1-cyclohexene-1-carboxylic acid. When methyl 3,3-dimethyl-2-(2-methyl-1-propenyl)cyclopropanecarboxylate is used as the chrysanthemic acid compound (8), at least one oxygen-containing compound selected from methyl 3,3-dimethyl-2-(2-E-hydroxymethyl-1-propenyl)cyclopropanecarboxylate, methyl 3,3-dimethyl-2-(2-E-formyl-1-propenyl)cyclopropanecarboxylate and methyl 3,3-dimethyl-2-(2-carboxy-1-propenyl)cyclopropanecarboxylate.

The amount of the oxidation catalyst composition to be used is usually 0.01 to 0.95 mole relative to 1 mole of the olefin compound in terms of selenium.

Examples of the organic hydroperoxide include tert-butyl hydroperoxide, tert-amyl hydroperoxide, cumene hydroperoxide and cymene hydroperoxide. The organic hydroperoxide is usually used in a form of an aqueous solution or an organic solvent solution. A concentration of the organic hydroperoxide in the aqueous solution or the organic solvent solution is not particularly limited, and taking into consideration volume efficiency and safety, it is practically 1 to 90% by weight. As the organic hydroperoxide, a commercially available organic hydroperoxide may be used as it is and it may be used by appropriately adjusting the concentration by dilution or concentration.

The amount of the organic hydroperoxide to be used may be decided suitably according to the desired oxygen-containing compound since the kind and the producing ratio of the obtained oxygen-containing compounds differs depending on the amount of the organic hydroperoxide to be used. For example, when the olefin compound (1) is used, the alcohol compound (2) is usually produced mainly by using 1 to 1.5 moles of the organic hydroperoxide relative to 1 mole of the olefin compound (1). The ketone compound (3) is usually produced mainly by using more than 1.5 moles of the organic hydroperoxide relative to 1 mole of the olefin compound (1). In this case, there is no specific upper limit of the amount of the organic hydroperoxide to be used, and the practical amount thereof is 50 moles or less.

When the olefin compound (4) is used, the alcohol compound (5) is usually produced mainly by using 1 to 2 moles of the organic hydroperoxide relative to 1 mole of the olefin compound (4), and the aldehyde compound (6) is usually produced mainly by using 2 to 3.5 moles of the organic hydroperoxide relative to 1 mole of the olefin compound (4). The carboxylic acid compound (7) is usually produced mainly by using more than 3.5 moles of the organic hydroperoxide relative to 1 mole of the olefin compound (4). In this case, there is no specific upper limit of the amount of the organic hydroperoxide to be used, and the practical amount thereof is 50 moles or less.

The reaction of the olefin compound and the organic compound is usually carried out in the presence of a solvent. Examples of the solvent include ether solvents such as diethyl ether, methyl tert-butyl ether and tetrahydrofuran; ester solvents such as ethyl acetate; alcohol solvents such as tert-butanol; halogenated hydrocarbon solvents such as chloroform, dichloromethane and chlorobenzene; nitrile solvents such as acetonitrile and propionitrile; water; and an ionic liquid. These solvents may be used alone or in the form of a mixture. Water, the ionic liquid and the mixed solvent of water and the ionic liquid are preferable and in view of improving the oxidation catalytic activity to be able to carry out the reaction more efficiently, the ionic liquid and the mixed solvent of water and the ionic liquid are more preferable. The amount of the solvent to be used is not particularly limited. When an oxidation catalyst composition containing the solvent is used for the present reaction as it is, the solvent may be not added.

The reaction of the olefin compound and the organic hydroperoxide is usually carried out by mixing the olefin compound, the organic hydroperoxide, the oxidation catalyst composition and the solvent, and the mixing order is not particularly limited and it is preferred to mixing the oxidation catalyst composition and the solvent followed by adding the olefin compound and the organic hydroperoxide thereto.

The reaction may be carried out while preparing the oxidation catalyst composition in the reaction system by adding the selenium compound, the nitrogen-containing aromatic compound and the acid to the reaction system in place of the oxidation catalyst composition preliminarily prepared.

The reaction temperature is usually 0 to 200°C. The reaction may be carried out under ordinary pressure conditions, and may be carried out under pressurized conditions. The progress of the reaction can be confirmed by a conventional analytical means such as gas chromatography, high performance liquid chromatography, thin layer chromatography, NMR and IR.

After completion of the reaction, an aqueous layer containing the oxidation catalyst composition and an organic layer containing the oxygen-containing compound can be separated by, if necessary after adding water and a hydrophobic solvent to the reaction liquid, conducting a separation treatment. The oxygen-containing compound can be isolated by distilling away the solvent from the organic layer obtained. The organic hydroperoxide remaining in the reaction liquid may be decomposed with a reducing agent such as sodium sulfite before conducting the separation treatment. The aqueous layer containing the oxidation catalyst composition can be reused in the present reaction, if necessary after concentrating and in this case, the other component such as the selenium compound may be added thereto.

Examples of the hydrophobic solvent include an aliphatic hydrocarbon solvent such as pentane, hexane and heptane; an aromatic hydrocarbon solvent such as toluene and xylene; an ether solvent such as diethyl ether and methyl tert-butyl ether; an ester solvent such as ethyl acetate; and a halogenated hydrocarbon solvent such as chloroform, dichloromethane and chlorobenzene. The amount thereof to be used is not particularly limited.

When the ionic liquid is used as the solvent, the ionic liquid layer and the organic layer containing oxygen-containing compound can be separated by using an ionic liquid nonmiscible solvent in place of the hydrophobic solvent. The ionic liquid layer includes the oxidation catalyst composition and the ionic liquid layer can be recycled to the present reaction, if necessary after concentration. In this case, the selenium compound may be added thereto, if necessary. Examples of the ionic liquid nonmiscible solvent include an aliphatic hydrocarbon solvent such as pentane, hexane and heptane; and an aromatic hydrocarbon solvent such as toluene and xylene.

Among the oxygen-containing compounds thus obtained, examples of the alcohol compound (2) or the alcohol compound (5) include E,E-2,6-dimethyl-8-acetoxy-2,6-octadien-1-ol, E,E-2,6-dimethyl-8-benzoyloxy-2,6-octadien-1-ol, E,E-2,6-dimethyl-8-methoxy-2,6-octadien-1-ol, E,E-2,6-dimethyl-8-benzyloxy-2,6-octadien-1-ol, E,E-2,6-dimethyl-2,6-octadien-1-ol-8-phenylsulfone, 1-hexen-3-ol, 1-hepten-3-ol, 1-octen-3-ol, 1-dodecen-3-ol, 1-hydroxy-2-cyclopentene, 1-hydroxy-2-cyclohexene, 1-hydroxy-2-cycloheptene, 1-hydroxy-2-cyclooctene, 1-hydroxy-4-methyl-2-cyclopentene, 1-hydroxy-5-methyl-2-cyclopentene, 1-hydroxy-4,5-dimethyl-2-cyclopentene, 1-hydroxy-4-chloro-2-cyclopentene, 1-hydroxy-4-methyl-2-cyclohexene, 4-hydroxy-2-hexene, 3-hydroxy-1,7-octadiene, 3-hydroxy-1,2,3,6-tetrahydrophthalic anhydride, 1-inden-1-ol, 2-phenyl-2-propen-1-ol, 2-methylenecyclobutanol, 2-methylenecyclopentanol, pinocarveol, dihydro-4-hydroxy-3-methylene-2-furanone, 2-cyclohexylidenecyclohexanol, 2-(2-hydroxy-1-methylmethylidene)-5-methylcyclohexanone, 3-hydroxymethyl-5,5-dimethyl-2-cyclohexen-1-one, 2-caren-10-ol, isochamol and myrthenol.

Examples of the ketone compound (3) include 3-oxo-1-hexene, 3-oxo-1-heptene, 3-oxo-1-octene, 3-oxo-1-dodecene, 2-cyclopentenone, 2-cyclohexenone, 2-cycloheptenone, 2-cyclooctenone, 4-methyl-2-cyclopentenone, 5-methyl-2-cyclopentenone, 4,5-dimethyl-2-cyclopentenone, 4-chloro-2-cyclopentenone, 4-methyl-2-cyclohexenone, 4-oxo-2-hexene, 3-oxo-1,7-octadiene, 3-oxo-1,2,3,6-tetrahydrophthalic anhydride, inden-1-one, 2-methylenecyclobutanone, 2-methylenecyclopentanone, pinocarvone, dihydro-4-oxo-3-methylene-2-furanone and 2-cyclohexylidenecyclohexanone.

Examples of the aldehyde compound (6) include E,E-2-formyl-8-acetoxy-6-methyl-2,6-octadiene, E,E-2-formyl-8-benzoyloxy-6-methyl-2,6-octadiene, E,E-2-formyl-8-methoxy-6-methyl-2,6-octadiene, E,E-2-formyl-8-benzyloxy-6-methyl-2,6-octadiene, E,E-2-formyl-8-phenylsulfone-6-methyl-2,6-octadiene, α-formylstyrene, 2-(4-methyl-2-cyclohexylidene)propanal, formylisophorone, 7,7-dimethyl-2-norcarene-3-carboxyaldehyde, 7,7-dimethyl-2-norcarene-3-carboxyaldehyde and myrthenal.

Examples of the carboxylic acid compound (7) include E,E-2-carboxy-8-acetoxy-6-methyl-2,6-octadiene, E,E-2-carboxy-8-benzoyloxy-6-methyl-2,6-octadiene, E,E-2-carboxy-8-methoxy-6-methyl-2,6-octadiene, E,E-2-carboxy-8-benzyloxy-6-methyl-2,6-octadiene, E,E-2-carboxy-8-phenylsulfone-6-methyl-2,6-octadiene, a-carboxystyrene, 2-(4-methyl-2-cyclohexylidene)propanoic acid, 5,5-dimethyl-3-oxo-1-cyclohexene-1-carboxylic acid, 7,7-dimethyl-bicyclo[4.1.0]hept-2-ene-3-carboxylic acid, caminic acid and myrthenic acid.

Examples of the alcohol compound (9) include 3,3-dimethyl-2-(2-E-hydroxymethyl-1-propenyl)cyclopropanecarboxylic acid, methyl 3,3-dimethyl-2-(2-E-hydroxymethyl-1-propenyl)cyclopropanecarboxylate, ethyl 3,3-dimethyl-2-(2-E-hydroxymethyl-1-propenyl)cyclopropanecarboxylate, isopropyl 3,3-dimethyl-2-(2-E-hydroxymethyl-1-propenyl)cyclopropanecarboxylate, tert-butyl 3,3-dimethyl-2-(2-E-hydroxymethyl-1-propenyl)cyclopropanecarboxylate, cyclohexyl 3,3-dimethyl-2-(2-E-hydroxymethyl-1-propenyl)cyclopropanecarboxylate, menthyl 3,3-dimethyl-2-(2-E-hydroxymethyl-1-propenyl)cyclopropanecarboxylate, benzyl 3,3-dimethyl-2-(2-E-hydroxymethyl-1-propenyl)cyclopropanecarboxylate, 4-chlorobenzyl 3,3-dimethyl-2-(2-E-hydroxymethyl-1-propenyl)cyclopropanecarboxylate, 2,3,5,6-tetrafluorobenzyl 3,3-dimethyl-2-(2-E-hydroxymethyl-1-propenyl)cyclopropanecarboxylate, 2,3,5,6-tetrafluoro-4-methylbenzyl 3,3-dimethyl-2-(2-E-hydroxymethyl-1-propenyl)cyclopropanecarboxylate, 2,3,5,6-tetrafluoro-4-methoxybenzyl 3,3-dimethyl-2-(2-E-hydroxymethyl-1-propenyl)cyclopropanecarboxylate, 2,3,5,6-tetrafluoro-4-methoxymethylbenzyl 3,3-dimethyl-2-(2-E-hydroxymethyl-1-propenyl)cyclopropanecarboxylate and 3-phenoxybenzyl 3,3-dimethyl-2-(2-E-hydroxymethyl-1-propenyl)cyclopropanecarboxylate.

Examples of the aldehyde compound (10) include 3,3-dimethyl-2-(2-E-formyl-1-propenyl)cyclopropanecarboxylic acid, methyl 3,3-dimethyl-2-(2-E-formyl-1-propenyl)cyclopropanecarboxylate, ethyl 3,3-dimethyl-2-(2-E-formyl-1-propenyl)cyclopropanecarboxylate, isopropyl 3,3-dimethyl-2-(2-E-formyl-1-propenyl)cyclopropanecarboxylate, tert-butyl 3,3-dimethyl-2-(2-E-formyl-1-propényl)cyclopropanecarboxylate, cyclohexyl 3,3-dimethyl-2-(2-E-formyl-1-propenyl)cyclopropanecarboxylate, menthyl 3,3-dimethyl-2-(2-E-formyl-1-propenyl)cyclopropanecarboxylate, benzyl 3,3-dimethyl-2-(2-E-formyl-1-propenyl)cyclopropanecarboxylate, 4-chlorobenzyl 3,3-dimethyl-2-(2-E-formyl-1-propenyl)cyclopropanecarboxylate, 2,3,5,6-tetrafluorobenzyl 3,3-dimethyl-2-(2-E-formyl-1-propenyl)cyclopropanecarboxylate, 2,3,5,6-tetrafluoro-4-methylbenzyl 3,3-dimethyl-2-(2-E-formyl-1-propenyl)cyclopropanecarboxylate, 2,3,5,6-tetrafluoro-4-methoxybenzyl 3,3-dimethyl-2-(2-E-formyl-1-propenyl)cyclopropanecarboxylate, 2,3,5,6-tetrafluoro-4-methoxymethylbenzyl 3,3-dimethyl-2-(2-E-formyl-1-propenyl)cyclopropanecarboxylate and 3-phenoxybenzyl 3,3-dimethyl-2-(2-E-formyl-1-propenyl)cyclopropanecarboxylate.

Examples of the carboxylic acid compound (11) include 3,3-dimethyl-2-(2-E-carboxy-1-propenyl)cyclopropanecarboxylic acid, methyl 3,3-dimethyl-2-(2-E-carboxy-1-propenyl)cyclopropanecarboxylate, ethyl 3,3-dimethyl-2-(2-E-carboxy-1-propenyl)cyclopropanecarboxylate, isopropyl 3,3-dimethyl-2-(2-E-carboxy-1-propenyl)cyclopropanecarboxylate, tert-butyl 3,3-dimethyl-2-(2-E-carboxy-1-propenyl)cyclopropanecarboxylate, cyclohexyl 3,3-dimethyl-2-(2-E-carboxy-1-propenyl)cyclopropanecarboxylate, menthyl 3,3-dimethyl-2-(2-E-carboxy-1-propenyl)cyclopropanecarboxylate, benzyl 3,3-dimethyl-2-(2-E-carboxy-1-propenyl)cyclopropanecarboxylate, 4-chlorobenzyl 3,3-dimethyl-2-(2-E-carboxy-1-propenyl)cyclopropanecarboxylate, 2,3,5,6-tetrafluorobenzyl 3,3-dimethyl-2-(2-E-carboxy-1-propenyl)cyclopropanecarboxylate, 2,3,5,6-tetrafluoro-4-methylbenzyl 3,3-dimethyl-2-(2-E-carboxy-1-propenyl)cyclopropanecarboxylate, 2,3,5,6-tetrafluoro-4-methoxybenzyl 3,3-dimethyl-2-(2-E-carboxy-1-propenyl)cyclopropanecarboxylate, 2,3,5,6-tetrafluoro-4-methoxymethylbenzyl 3,3-dimethyl-2-(2-E-carboxy-1-propenyl)cyclopropanecarboxylate and 3-phenoxybenzyl 3,3-dimethyl-2-(2-E-carboxy-1-propenyl)cyclopropanecarboxylate.

When a trans-isomer thereof is used as the chrysanthemic acid compound (8), trans-isomer of the oxygen-containing compound is usually obtained. When a cis-isomer thereof is used as the chrysanthemic acid compound (8), cis-isomer of the oxygen-containing compound is usually obtained. When an optically active chrysanthemic acid compound (8) is used, an optically active oxygen-containing compound is usually obtained.

Next, the reaction of the cycloalkanone compound and hydrogen peroxide is illustrated.

The cycloalkanone compound may be a compound wherein the compound has a cycloalkane skelton and at least one carbon atom forming the cycloalkanone skelton is a carbonyl group. Examples thereof include a cycloalkanone compound represented by the formula (12) (hereinafter, simply referred to as the cycloalkanone compound (12)): 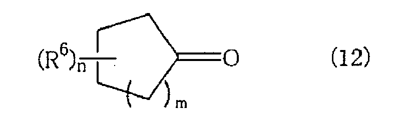 wherein R⁶ represents a hydrogen atom; a halogen atom; a C1-C20 alkyl group; a C6-C10 aryl group; a C7-C12 aralkyl group; or a C1-C20 alkoxy group, and herein, the above-mentioned C1-C20 alkyl group, C6-C10 aryl group, C7-C12 aralkyl group and C1-C20 alkoxy group are optionally substituted with a halogen atom or atoms, or a C1-C20 alkoxy group or groups, m represents an integer of 0 to 8, n represents an integer of 0 to 11 which satisfies n ≦m+3, and herein, when n is 2 or more, R⁶ may be the same or different, and any of two R⁶ may be bonded to form a ring.

Examples of the halogen atom, the C1-C20 alkyl group, the C6-C10 aryl group, the C7-C12 aralkyl group and the C1-C20 alkoxy group include those as same as described above.

Examples of the cycloalkanone compound (12) include cyclobutanone, 3-methylcyclobutanone, 3-phenylcyclobutanone, 3-chlorocyclobutanone, cyclopentanone, 3-methylcyclopentanone, 3-phenylcyclopentanone, 3-chlorocyclopentanone, cyclohexanone, 4-methylcyclohexanone, 3-methylcyclohexanone, 2-methylcyclohexanone, 4-methoxycyclohexanone, 4-tert-butylcyclohexanone, 4-phenylcyclohexanone, 4-chlorocyclohexanone, cycloheptanone, 4-methylcycloheptanone, 4-phenylcycloheptanone, 3-chlorocycloheptanone, cyclooctanone, cyclononanone, cyclodecanone, cyclododecanone, 10-methyl-2-decalone, 2-decalone and hexahydro-2-indanone. The cycloalkanone compound having two or more carbonyl groups within a molecule such as 1,3-cyclopentandione and 1,4-cyclohexanedione are also exemplified.

A commercially available cycloalkanone compound may be used and that produced according to a known method comprising oxidation of the corresponding cycloalkanol compound.

The cycloalkanecarboxylic acid compound is obtained as the oxygen-containing compound by reacting the cycloalkanone compound with hydrogen peroxide in the presence of the oxidation catalyst composition, and the ring of the cycloalkanecarboxylic acid compound obtained is usually composed of carbon atoms which numbers are one fewer than number of carbon atoms composing the ring of the cycloalkanone compound. When two carbon atoms are carbonyl groups among carbon atoms composing the ring of the cycloalkanone compound, the cyloalkanecarboxylic acid having the ring composed of carbon atoms which numbers are two fewer than number of carbon atoms composing the ring of the cycloalkanone compound is sometimes obtained depending on the reaction condition.

When the cycloalkanone compound (12) is used, a cycloalkanecarboxylic acid compound represented by the formula (13) (hereinafter, simply referred to as the cycloalkanecarboxylic acid compound (13)): 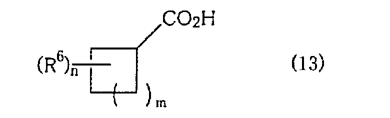 wherein R⁶, m and n are the same as the described above, is obtained as the oxygen-containing compound.

The amount of the oxidation catalyst composition to be used is usually 0.001 to 0.95 mole relative to 1 mole of the cycloalkanone compound based on selenium.

An aqueous hydrogen peroxide solution may be used and a solution of hydrogen peroxide in an organic solvent may be used. It is preferred to use the aqueous hydrogen peroxide solution. The concentration of hydrogen peroxide in the aqueous hydrogen peroxide solution or in the solution of hydrogen peroxide in the organic solvent is not particularly limited, but in view of volume efficacy and safety, the concentration is practically 1 to 60% by weight. A commercially available aqueous hydrogen peroxide solution may be used as it is, and it may be used after adjusting the concentration by dilution or concentration.

The amount of hydrogen peroxide to be used is usually 1 mole or more relative to 1 mole of the cycloalkanone compound. There is no specific upper limit and in view of economic point, it is practically 10 moles or less.

The reaction of the cycloalkanone compound and hydrogen peroxide may be carried out in absence of a solvent and in presence of the solvent. Examples of the solvent include an ether solvent such as diethyl ether, methyl tert-butyl ether and tetrahydrofuran; an ester solvent such as ethyl acetate; an alcohol solvent such as tert-butanol; a halogenated hydrocarbon solvent such as chloroform, dichloromethane and chlorobenzene; a nitrile solvent such as acetonitrile and propionitrile; water; an ionic liquid; and a mixture thereof. Among them, water, the ionic liquid or a mixed solvent of water and the ionic liquid is preferable. The amount thereof to be used is not limited.

When an oxidation catalyst composition containing the solvent is used as it is, the solvent may be not added.

The reaction of the cycloalkanone compound and hydrogen peroxide is carried out by mixing the cycloalkanone compound, hydrogen peroxide, the oxidation catalyst composition and, if necessary, the solvent, and the mixing order is not particularly limited.

The reaction may be carried out while preparing the oxidation catalyst composition in the reaction system by adding the selenium compound, the nitrogen-containing aromatic compound and the acid to the reaction system in place of the oxidation catalyst composition preliminarily prepared.

The reaction temperature is usually 0 to 200°C. The reaction may be carried out under ordinary pressure conditions, and may be carried out under pressurized conditions. The progress of the reaction can be confirmed by a conventional analytical means such as gas chromatography, high performance liquid chromatography, thin layer chromatography, NMR and IR.

After completion of the reaction, an aqueous layer containing the oxidation catalyst composition and an organic layer containing the cycloalkanecarboxylic acid compound can be separated by, if necessary after adding water and a hydrophobic solvent to the reaction liquid, conducting a separation treatment. The cycloalkanecarboxylic acid compound can be isolated by distilling away the solvent from the organic layer obtained. The organic hydroperoxide remaining in the reaction liquid may be decomposed with a reducing agent such as sodium sulfite before conducting the separation treatment. The aqueous layer containing the oxidation catalyst composition can be reused in the present reaction, if necessary after concentrating and in this case, the other component such as the selenium compound may be added thereto.

Examples of the cycloalkanecarboxylic acid compound (13) thus obtained include cyclopropanecarboxylic acid, 2-methylcyclopropanecarboxylic acid, 2-phenylcyclopropanecarboxylic acid, 2-chlorocyclopropanecarboxylic acid, cyclobutanecarboxylic acid, 2-methylcyclobutanecarboxylic acid, 2-phenylcyclobutanecarboxylic acid, 2-chlorocyclobutanecarboxylic acid, cyclopentanecarboxylic acid, 3-methylcyclopentanecarboxylic acid, 2-methylcyclopentanecarboxylic acid, 3-methoxycyclopentanecarboxylic acid, 3-tert-butylcyclopentanecarboxylic acid, 3-phenylcyclopentanecarboxylic acid, 3-chlorocyclopentanecarboxylic acid, cyclohexanecarboxylic acid, 3-methylcyclohexanecarboxylic acid, 4-methylcyclohexanecarboxylic acid, 3-phenylcyclohexanecarboxylic acid, 4-phenylcyclohexanecarboxylic acid, 2-chlorocyclohexanecarboxylic acid, cycloheptanecarboxylic acid, cyclooctanecarboxylic acid, cyclononanecarboxylic acid, cyclodecanecarboxylic acid, 10-methylhexahydro-2-indanecarboxylic acid, hexahydro-2-indanecarboxylic acid and bicycle[4.2.0]octane-7-carboxylic acid.

Examples of the cycloalkanecarboxylic acid compound obtained in the case of using the cycloalkanone compound having two or more carbonyl groups within a molecule such as 1,3-cyclopentandione and 1,4-cyclohexanedione include 2-oxocyclobutanecarboxylic acid and 3-oxocyclopentanecarboxylic acid.

### Examples

In the following Examples, hydroxyester, formylester and carboxyester respectively means methyl 3,3-dimethyl-2-(2-E-hydroxymethyl-1-propenyl)cyclopropanecarboxylate, methyl 3,3-dimethyl-2-(2-E-formyl-1-propenyl)cyclopropanecarboxylate and methyl 3,3-dimethyl-2-(2-E-carboxy-1-propenyl)cyclopropanecarboxylate. Hydroxycarboxylic acid, formylcarboxylic acid and carboxycarboxylic acid respectively means 3,3-dimethyl-2-(2-E-hydroxymethyl-1-propenyl)cyclopropanecarboxylic acid, 3,3-dimethyl-2-(2-E-formyl-1-propenyl)cyclopropanecarboxylic acid and 3,3-dimethyl-2-(2-E-carboxy-1-propenyl)cyclopropanecarboxylic acid.

The yields of hydroxyester, hydroxycarboxylic acid, carboxyester and carboxycarboxylic acid were calculated by the high performance liquid chromatography internal standard method and the yields of the others were calculated by the gas chromatography internal standard method.

### Example 1

Into a 5 ml sample bottle, 100 mg of selenium dioxide, 444 mg of N-methylimidazole and 1130 mg of 42% by weight aqueous tetrafluoroboric acid solution were charged and the resultant mixture was stirred at room temperature for 10 minutes to obtain a homogeneous solution containing a selenium-containing oxidation catalyst composition. 800 mg of the homogeneous solution was taken and ⁷⁷Se-NMR spectrum thereof was measured to observe a Se peak at 1309.6 ppm (reference material: dimethyl selenide).

Into a 50 ml flask, 800 mg of the homogeneous solution containing a selenium-containing oxidation catalyst composition obtained was charged. 90 mg of cyclopentanone and 30% by weight hydrogen peroxide were added thereto and the reaction was conducted at room temperature for 4 hours. To the reaction mixture, 10 g of ethyl acetate was added and then the mixture was separated to two layers. The ethyl acetate layer was analyzed by the gas chromatography internal standard method to find that the yield of cyclobutanecarboxylic acid was 16% and the raw material, cyclopentanone, was remained in 18%.

### Example 2

Into a 5 ml sample bottle, 70 mg of selenium dioxide, 300 mg of pyridine and 791 mg of 42% by weight aqueous tetrafluoroboric acid solution were charged and the resultant mixture was stirred at room temperature for 10 minutes to obtain slurry in which white crystals were precipitated. To the slurry, 500 mg of water was added to obtain a homogeneous solution containing selenium-containing oxidation catalyst composition. 800 mg of the homogeneous solution was taken and ⁷⁷Se-NMR spectrum thereof was measured to observe a Se peak at 1308.5 ppm (reference material: dimethyl selenide).

### Example 3

Into a 5 ml sample bottle, 70 mg of selenium dioxide, 311 mg of N-methylimdazloe and 436 mg of 85% by weight phosphoric acid were charged and the resultant mixture was stirred at room temperature for 10 minutes to obtain slurry in which white crystals were precipitated. To the slurry, 500 mg of water was added to obtain a homogeneous solution containing selenium-containing oxidation catalyst composition. 800 mg of the homogeneous solution was taken and ⁷⁷Se-NMR spectrum thereof was measured to observe a Se peak at 1313.5 ppm (reference material: dimethyl selenide).

### Example 4

Into a 100 ml flask equipped with a magnetic stirrer and a reflux condenser, 90 mg of selenium dioxide, 560 mg of 2-ethyl-4-methylimidazole and 881 mg of 42% by weight aqueous tetrafluoroboric acid solution were charged and the resulting mixture was stirred at room temperature for 10 minutes to obtain a homogeneous solution containing selenium-containing oxidation catalyst composition. To the homogeneous solution, 850 mg of 70% by weight aqueous tert-butylhydroperoxide solution and 500 mg of methyl 3,3-dimethyl-2-(2-methyl-1-propenyl)cyclopropanecarboxylate were charged and the reaction was conducted at an inner temperature of 70°C for 3 hours. After competition of the reaction, the reaction mixture was cooled to room temperature and 10 g of hexane was added thereto and the mixture was separated to obtain a hexane layer and an aqueous layer containing the selenium-containing oxidation catalyst composition. The aqueous layer containing the selenium-containing oxidation catalyst composition was extracted twice with hexane and the organic layer obtained was mixed with the hexane layer obtained before to obtain a hexane layer containing oxygen-containing compounds.

### <Products and Yields thereof>

Formylester: 66%, carboxyester: 8%.

The content of selenium in the hexane layer was 10 ppm (measured by ICP emission method) and 99.3% of selenium used was recovered in the aqueous layer containing the selenium-containing oxidation catalyst composition.

### Example 5

Into a 100 ml flask equipped with a magnetic stirrer and a reflux condenser, the aqueous layer containing the selenium-containing oxidation catalyst composition obtained in Example 4, 850 mg of 70% by weight aqueous tert-butylhydroperoxide solution and 500 mg of methyl 3,3-dimethyl-2-(2-methyl-1-propenyl)cyclopropanecarboxylate were charged and the reaction was conducted at an inner temperature of 80°C for 3 hours. After competition of the reaction, the reaction mixture was cooled to room temperature and 10 g of hexane was added thereto and the mixture was separated to obtain a hexane layer and an aqueous layer containing the selenium-containing oxidation catalyst composition. The aqueous layer containing the selenium-containing oxidation catalyst composition was extracted twice with hexane and the organic layer obtained was mixed with the hexane layer obtained before to obtain a hexane layer containing oxygen-containing compounds.

### <Products and Yields thereof>

Hydroxyester: 10%, formylester: 76%, carboxyester: 13%.

The content of selenium in the hexane layer was 12 ppm (measured by ICP emission method) and 99.2% of selenium used was recovered in the aqueous layer containing the selenium-containing oxidation catalyst composition.

### Example 6

Into a 100 ml flask equipped with a magnetic stirrer and a reflux condenser, 90 mg of selenium dioxide, 690 mg of 3-butylpyridine and 881 mg of 42% by weight aqueous tetrafluoroboric acid solution were charged and the resulting mixture was stirred at room temperature for 10 minutes to obtain a homogeneous solution containing selenium-containing oxidation catalyst composition. To the homogeneous solution, 850 mg of 70% by weight aqueous tert-butylhydroperoxide solution and 500 mg of methyl 3,3-dimethyl-2-(2-methyl-1-propenyl)cyclopropanecarboxylate were charged and the reaction was conducted at an inner temperature of 70°C for 4 hours. After competition of the reaction, the reaction mixture was cooled to room temperature and 10 g of hexane was added thereto and the mixture was separated to obtain a hexane layer and an aqueous layer containing the selenium-containing oxidation catalyst composition. The aqueous layer containing the selenium-containing oxidation catalyst composition was extracted twice with hexane and the organic layer obtained was mixed with the hexane layer obtained before to obtain a hexane layer containing oxygen-containing compounds.

### <Products and Yields thereof>

Formylester: 79%, carboxyester: 6%.

The raw material, methyl 3,3-dimethyl-2-(2-methyl-1-propenyl)cyclopropanecarboxylate, was recovered in 5%.

### Example 7

Into a 300 ml flask equipped with a magnetic stirrer and a reflux condenser, 1.8 g of selenium dioxide, 8.3 g of N-methylimidazole and 17.6 g of 42% by weight aqueous tetrafluoroboric acid solution were charged and the resulting mixture was stirred at room temperature for 30 minutes to obtain a homogeneous solution containing selenium-containing oxidation catalyst composition. To the homogeneous solution, 4 g of 70% by weight aqueous tert-butylhydroperoxide solution and 10 g of methyl 3,3-dimethyl-2-(2-methyl-1-propenyl)cyclopropanecarboxylate were charged and the resultant mixture was adjusted at an inner temperature of 50°C. 14 g of 70% by weight aqueous tert-butylhydroperoxide solution was added thereto dropwise over 3 hours and then the reaction was conducted at the same temperature for 21 hours. After competition of the reaction, the reaction mixture was cooled to room temperature and 100 g of hexane was added thereto and the mixture was separated to obtain a hexane layer and an aqueous layer containing the selenium-containing oxidation catalyst composition. The aqueous layer containing the selenium-containing oxidation catalyst composition was extracted twice with hexane and the organic layer obtained was mixed with the hexane layer obtained before to obtain a hexane layer containing oxygen-containing compounds.

### <Products and Yields thereof>

Hydroxyester: 12%, formyl: 67%, carboxyester: 16%. The raw material, methyl 3,3-dimethyl-2-(2-methyl-1-propenyl)cyclopropanecarboxylate, was recovered in 3%.

### Example 8

According to the same manner as that described in Example 7, a hexane layer containing oxygen-containing compounds and an aqueous layer containing the selenium-containing oxidation catalyst composition were obtained except that the aqueous layer containing the selenium-containing oxidation catalyst composition obtained in Example 7 was used in place of the homogeneous solution containing selenium-containing oxidation catalyst composition and the reaction time was 33 hours.

### <Products and Yields thereof>

Hydroxyester: 4%, formylester: 80%, carboxyester: 11%. The raw material, methyl 3,3-dimethyl-2-(2-methyl-1-propenyl)cyclopropanecarboxylate, was recovered in 2%.

### Example 9

According to the same manner as that described in Example 7, a hexane layer containing oxygen-containing compounds and an aqueous layer containing the selenium-containing oxidation catalyst composition were obtained except that the aqueous layer containing the selenium-containing oxidation catalyst composition obtained in Example 8 was used in place of the homogeneous solution containing selenium-containing oxidation catalyst composition and the reaction time was 33 hours.

### <Products and Yields thereof>

Hydroxyester: 7%, formylester: 75%, carboxyester: 13%. The raw material, methyl 3,3-dimethyl-2-(2-methyl-1-propenyl)cyclopropanecarboxylate, was recovered in 2%.

### Example 10

According to the same manner as that described in Example 7, a hexane layer containing oxygen-containing compounds and an aqueous layer containing the selenium-containing oxidation catalyst composition were obtained except that the aqueous layer containing the selenium-containing oxidation catalyst composition obtained in Example 9 was used in place of the homogeneous solution containing selenium-containing oxidation catalyst composition and the reaction time was 33 hours.

### <Products and Yields thereof>

Hydroxyester: 6%, formylester: 78%, carboxyester: 11%. The raw material, methyl 3,3-dimethyl-2-(2-methyl-1-propenyl)cyclopropanecarboxylate, was recovered in 1%.

### Example 11

Into a 300 ml flask equipped with a magnetic stirrer and a reflux condenser, 1.8 g of selenium dioxide, 6 g of pyridine, 18 g of N-methyl-4-butylpyridinium tetrafluoroborate and 10 g of 42% by weight tetrafluoroboric acid solution were charged and the resulting mixture was stirred at room temperature for 30 minutes to obtain a homogeneous selenium-containing oxidation catalyst composition solution. To the selenium-containing oxidation catalyst composition solution, 4 g of 70% by weight aqueous tert-butylhydroperoxide solution and 10 g of methyl 3,3-dimethyl-2-(2-methyl-1-propenyl)cyclopropanecarboxylate were charged and the resultant mixture was adjusted at an inner temperature of 50°C. 14 g of 70% by weight aqueous tert-butylhydroperoxide solution was added thereto dropwise over 3 hours and then the reaction was conducted at the same temperature for 7 hours. After competition of the reaction, the reaction mixture was cooled to room temperature and 100 g of hexane was added thereto and the mixture was separated to obtain an aqueous layer containing the selenium-containing oxidation catalyst composition and the ionic liquid, and a hexane layer. The aqueous layer containing the selenium-containing oxidation catalyst composition and the ionic liquid was extracted twice with hexane and the organic layer obtained was mixed with the hexane layer obtained before to obtain a hexane layer containing oxygen-containing compounds.

### <Products and Yields thereof>

Hydroxyester: 26%, formylester: 64%, carboxyester: 10%.

### Example 12

According to the same manner as that described in Example 11, an aqueous layer containing the selenium-containing oxidation catalyst composition and the ionic liquid and a hexane layer containing oxygen-containing compounds were obtained except that the aqueous layer containing the selenium-containing oxidation catalyst composition and the ionic liquid obtained in Example 11 was used in place of the selenium-containing oxidation catalyst composition solution.

### <Products and Yields thereof>

Hydroxyester: 9%, formylester: 84%, carboxyester: 2%. The raw material, methyl 3,3-dimethyl-2-(2-methyl-1-propenyl)cyclopropanecarboxylate, was recovered in 3%.

### Example 13

According to the same manner as that described in Example 11, an aqueous layer containing the selenium-containing oxidation catalyst composition and the ionic liquid and a hexane layer containing oxygen-containing compounds were obtained except that the aqueous layer containing the selenium-containing oxidation catalyst composition and the ionic liquid obtained in Example 12 was used in place of the selenium-containing oxidation catalyst composition solution.

### <Products and Yields thereof>

Hydroxyester: 11%, formylester: 81%, carboxyester: 8%.

### Example 14

Into a 100 ml flask equipped with a magnetic stirrer and a reflux condenser, 25 mg of selenium dioxide, 160 mg of benzimidazole, 1 g of 1-butyl-3-methylimidaxolium tetrafluoroborate and 210 mg of 42% by weight tetrafluoroboric acid solution were charged and the resulting mixture was stirred at room temperature for 10 minutes to obtain a homogeneous selenium-containing oxidation catalyst composition solution. To the selenium-containing oxidation catalyst composition solution, 2.5 g of 70% by weight aqueous tert-butylhydroperoxide solution and 1.5 g of methyl 3,3-dimethyl-2-(2-methyl-1-propenyl)cyclopropanecarboxylate were charged and the reaction was conducted at an inner temperature of 60°C for 1 hour and further at an inner temperature of 80°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid, and a hexane layer. The aqueous layer containing the selenium-containing oxidation catalyst composition and the ionic liquid was extracted twice with hexane and the organic layer obtained was mixed with the hexane layer obtained before to obtain a hexane layer containing oxygen-containing compounds.

### <Products and Yields thereof>

Hydroxyester: 31%, formylester: 41%, carboxyester: 15%. The raw material, methyl 3,3-dimethyl-2-(2-methyl-1-propenyl)cyclopropanecarboxylate, was recovered in 10%.

### Example 15

According to the same manner as that described in Example 14, a hexane layer containing oxygen-containing compounds was obtained except that 85 mg of imidazole was used in place of 160 mg of benzimidazole.

### <Products and Yields thereof>

Hydroxyester: 34%, formylester: 41%, carboxyester: 9%. The raw material, methyl 3,3-dimethyl-2-(2-methyl-1-propenyl)cyclopropanecarboxylate, was recovered in 12%.

### Example 16 (does not fall within the scope of the present invention)

Into a 100 ml flask equipped with a magnetic stirrer and a reflux condenser, 25 mg of selenium dioxide, 125 mg of benzimidazole, 1 g of 1-butyl-3-methylimidaxolium tetrafluoroborate and 110 mg of 65% by weight nitric acid were charged and the resulting mixture was stirred at room temperature for 10 minutes to obtain a homogeneous selenium-containing oxidation catalyst composition solution. To the selenium-containing oxidation catalyst composition solution, 2.5 g of 70% by weight aqueous tert-butylhydroperoxide solution and 1.5 g of methyl 3,3-dimethyl-2-(2-methyl-1-propenyl)cyclopropanecarboxylate were charged and the reaction was conducted at an inner temperature of 60°C for 1 hour and further at an inner temperature of 70°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid, and a hexane layer. The aqueous layer containing the selenium-containing oxidation catalyst composition and the ionic liquid was extracted twice with hexane and the organic layer obtained was mixed with the hexane layer obtained before to obtain a hexane layer containing oxygen-containing compounds.

### <Products and Yields thereof>

Hydroxyester: 25%, formylester: 33%, carboxyester: 7%. The raw material, methyl 3,3-dimethyl-2-(2-methyl-1-propenyl)cyclopropanecarboxylate, was recovered in 30%.

### Example 17

According to the same manner as that described in Example 16, a hexane layer containing oxygen-containing compounds was obtained except that 130 mg of 85% by weight phosphoric acid was used in place of 110 mg of 65% by weight nitric acid.

### <Products and Yields thereof>

Hydroxyester: 34%, formylester: 32%, carboxyester: 8%. The raw material, methyl 3,3-dimethyl-2-(2-methyl-1-propenyl)cyclopropanecarboxylate, was recovered in 20%.

### Example 18

Into a 100 ml flask equipped with a magnetic stirrer and a reflux condenser, 140 mg of sodium selenite, 300 mg of pyridine, 900 mg of 1-butyl-3-methylimidaxolium tetrafluoroborate and 840 mg of 45% by weight aqueous tetrafluoroboric acid solution were charged and the resulting mixture was stirred at room temperature for 10 minutes to obtain a homogeneous selenium-containing oxidation catalyst composition solution. To the selenium-containing oxidation catalyst composition solution, 900 mg of 70% by weight aqueous tert-butylhydroperoxide solution and 500 mg of methyl 3,3-dimethyl-2-(2-methyl-1-propenyl)cyclopropanecarboxylate were charged and the reaction was conducted at an inner temperature of 50°C for 6 hours. After competition of the reaction, the reaction mixture was cooled to room temperature and 10 g of hexane was added thereto and the mixture was separated to obtain an aqueous layer containing the selenium-containing oxidation catalyst composition and the ionic liquid, and a hexane layer. The aqueous layer containing the selenium-containing oxidation catalyst composition and the ionic liquid was extracted twice with hexane and the organic layer obtained was mixed with the hexane layer obtained before to obtain a hexane layer containing oxygen-containing compounds.

### <Products and Yields thereof>

Hydroxyester: 28%, formylester: 44%, carboxyester: 15%. The raw material, methyl 3,3-dimethyl-2-(2-methyl-1-propenyl)cyclopropanecarboxylate, was recovered in 10%.

### Example 19

Into a 100 ml flask equipped with a magnetic stirrer and a reflux condenser, 90 mg of selenium dioxide, 450 mg of benzimidazole, 900 mg of 1-butyl-3-methylimidaxolium tetrafluoroborate and 440 mg of 42% by weight aqueous tetrafluoroboric acid solution were charged and the resulting mixture was stirred at room temperature for 10 minutes to obtain a homogeneous selenium-containing oxidation catalyst composition solution. To the selenium-containing oxidation catalyst composition solution, 850 mg of 70% by weight aqueous tert-butylhydroperoxide solution and 500 mg of methyl 3,3-dimethyl-2-(2-methyl-1-propenyl)cyclopropanecarboxylate were charged and the reaction was conducted at an inner temperature of 60°C for 2 hours and further at an inner temperature of 70°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid, and a hexane layer. The aqueous layer containing the selenium-containing oxidation catalyst composition and the ionic liquid was extracted twice with hexane to obtain 2.1 g of an aqueous layer containing the selenium-containing oxidation catalyst composition and the ionic liquid, and an organic layer. The organic layer was mixed with the hexane layer obtained before to obtain a hexane layer containing oxygen-containing compounds.

### <Products and Yields thereof>

Hydroxyester: 4%, formylester: 81%, carboxyester: 6%.

### Example 20

Into a 100 ml flask equipped with a magnetic stirrer and a reflux condenser, 2.1 g of the aqueous layer containing the selenium-containing oxidation catalyst composition and the ionic liquid obtained in Example 19 was charged. 850 mg of 70% by weight aqueous tert-butylhydroperoxide solution and 500 mg of methyl 3,3-dimethyl-2-(2-methyl-1-propenyl)cyclopropanecarboxylate were charged thereto and the reaction was conducted at an inner temperature of 60°C for 2 hours and further at an inner temperature of 70°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid and a hexane layer. The aqueous layer containing the selenium-containing oxidation catalyst composition and the ionic liquid was extracted twice with hexane to obtain 2.1 g of an aqueous layer containing the selenium-containing oxidation catalyst composition and the ionic liquid, and an organic layer. The organic layer was mixed with the hexane layer obtained before to obtain a hexane layer containing oxygen-containing compounds.

### <Products and Yields thereof>

Hydroxyester: 2%, formylester: 79%, carboxyester: 7%.

### Example 21

Into a 100 ml flask equipped with a magnetic stirrer and a reflux condenser, 2.1 g of the aqueous layer containing the selenium-containing oxidation catalyst composition and the ionic liquid obtained in Example 20 was charged. 850 mg of 70% by weight aqueous tert-butylhydroperoxide solution and 500 mg of methyl 3,3-dimethyl-2-(2-methyl-1-propenyl)cyclopropanecarboxylate were charged thereto and the reaction was conducted at an inner temperature of 60°C for 2 hours and further at an inner temperature of 70°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid and a hexane layer. The aqueous layer containing the selenium-containing oxidation catalyst composition and the ionic liquid was extracted twice with hexane to obtain 2.2 g of an aqueous layer containing the selenium-containing oxidation catalyst composition and the ionic liquid, and an organic layer. The organic layer was mixed with the hexane layer obtained before to obtain a hexane layer containing oxygen-containing compounds.

### <Products and Yields thereof>

Formylester: 79%, carboxyester: 8%.

### Example 22

Into a 100 ml flask equipped with a magnetic stirrer and a reflux condenser, 2.1 g of the aqueous layer containing the selenium-containing oxidation catalyst composition and the ionic liquid obtained in Example 21 was charged. 850 mg of 70% by weight aqueous tert-butylhydroperoxide solution and 500 mg of methyl 3,3-dimethyl-2-(2-methyl-1-propenyl)cycloprapanecarboxylate were charged thereto and the reaction was conducted at an inner temperature of 60°C for 2 hours and further at an inner temperature of 70°C for 2 hours. After competition of the reaction, the reaction mixture was cooled to room temperature and 10 g of hexane was added thereto and the mixture was separated to obtain an aqueous layer containing the selenium-containing oxidation catalyst composition and the ionic liquid and a hexane layer. The aqueous layer containing the selenium-containing oxidation catalyst composition and the ionic liquid was extracted twice with hexane to obtain 2.2 g of an aqueous layer containing the selenium-containing oxidation catalyst composition and the ionic liquid, and an organic layer. The organic layer was mixed with the hexane layer obtained before to obtain a hexane layer containing oxygen-containing compounds.

### <Products and Yields thereof>

Hydroxyester: 3%, formylester: 80%, carboxyester: 8%.

### Comparative Example 1

According to the same manner as that described in Example 19 except that benzimidazole was not used, hydroxyester, formylester and carboxycester were not produced, although a raw material, methyl 3,3-dimethyl-2-(2-methyl-1-propenyl)cyclopropanecarboxylate, was disappeared.

### Example 23

According to the same manner as that described in Example 19, a hexane layer containing oxygen-containing compounds was obtained except that 750 mg of 1,10-phenanethroline was used in place of 450 mg of benzimidazole.

### <Products and Yields thereof>

Hydroxyester: 4%, formylester: 74%, carboxyester: 1%.

### Example 24

According to the same manner as that described in Example 19, a hexane layer containing oxygen-containing compounds was obtained except that 300 mg of pyridine was used in place of 450 mg of benzimidazole, and 900 mg of N-methyl-4-butylpyridinium tetrafluoroborate was used in place of 900 mg of 1-butyl-3-methylimidazolium tetrafluoroborate.

### <Products and Yields thereof>

Hydroxyester: 2%, formylester: 73%, carboxyester: 16%.

### Example 25

Into a 100 ml flask equipped with a magnetic stirrer and a reflux condenser, 90 mg of selenium dioxide, 300 mg of pyridine, 900 mg of 1-butyl-3-methylimidaxolium tetrafluoroborate and 500 mg of 42% by weight aqueous tetrafluoroboric acid solution were charged and the resulting mixture was stirred at room temperature for 10 minutes to obtain a homogeneous selenium-containing oxidation catalyst composition solution. To the selenium-containing oxidation catalyst composition solution, 900 mg of 70% by weight aqueous tert-butylhydroperoxide solution and 500 mg of 3,3-dimethyl-2-(2-methyl-1-propenyl)cyclopropanecarboxylic acid were charged and the reaction was conducted at an inner temperature of 50°C for 6 hours. After competition of the reaction, the reaction mixture was cooled to room temperature and 10 g of methyl tert-butyl ether was added thereto and the mixture was separated to obtain an aqueous layer containing the selenium-containing oxidation catalyst composition and the ionic liquid and a methyl tert-butyl ether layer. The aqueous layer containing the selenium-containing oxidation catalyst composition and the ionic liquid was extracted twice with methyl tert-butyl ether to obtain an organic layer and 2.1 g of an aqueous layer containing the selenium-containing oxidation catalyst composition and the ionic liquid. The organic layer was mixed with the methyl tert-butyl ether layer obtained before to obtain a methyl tert-buyl ether layer containing oxygen-containing compounds.

### <Products and Yields thereof>

Hydroxycarboxylic acid: 32%, formylcarboxylic acid: 52%, carboxycarboxylic acid: 3%.

### Example 26

Into a 100 ml flask equipped with a magnetic stirrer and a reflux condenser, 2.1 g of the aqueous layer containing the selenium-containing oxidation catalyst composition and the ionic liquid obtained in Example 25 was charged. 900 mg of 70% by weight aqueous tert-butylhydroperoxide solution and 500 mg of 3,3-dimethyl-2-(2-methyl-1-propenyl)cyclopropanecarboxylic acid were charged thereto and the reaction was conducted at an inner temperature of 50°C for 6 hours. After competition of the reaction, the reaction mixture was cooled to room temperature and 10 g of methyl tert-butyl ether was added thereto and the mixture was separated to obtain an aqueous layer containing the selenium-containing oxidation catalyst composition and the ionic liquid and a methyl tert-butyl ether layer. The aqueous layer containing the selenium-containing oxidation catalyst composition and the ionic liquid was extracted twice with methyl tert-butyl ether to obtain an organic layer and 2.2 g of an aqueous layer containing the selenium-containing oxidation catalyst composition and the ionic liquid. The organic layer was mixed with the methyl tert-butyl ether layer obtained before to obtain a methyl tert-buyl ether layer containing oxygen-containing compounds.

### <Products and Yields thereof>

Hydroxycarboxylic acid: 8%, formylcarboxylic acid: 71%, carboxycarboxylic acid: 2%.

### Example 27

Into a 100 ml flask equipped with a magnetic stirrer and a reflux condenser, 28 mg of selenium dioxide, 100 mg of pyridine and 210 mg of 42% by weight aqueous tetrafluoroboric acid solution were charged and the resulting mixture was stirred at room temperature for 10 minutes to obtain a homogeneous solution containing a selenium-containing oxidation catalyst composition. To the homogeneous solution, 3.2 g of 70% by weight aqueous tert-butylhydroperoxide solution, 1.82 g of methyl 3,3-dimethyl-2-(2-methyl-1-propenyl)cyclopropanecarboxylate and 10 g of tert-butanol were charged and the reaction was conducted at an inner temperature of 60°C for 8 hours. After competition of the reaction, the reaction mixture was cooled to room temperature and 10 g of hexane and 10 g of water were added thereto and the mixture was separated to obtain an aqueous layer containing the selenium-containing oxidation catalyst composition and a hexane layer. The aqueous layer containing the selenium-containing oxidation catalyst composition was extracted twice with hexane and the organic layer obtained was mixed with the hexane layer obtained before to obtain a hexane layer containing oxygen-containing compounds.

### <Products and Yields thereof>

Hydroxyester: 33%, formylester: 25%, carboxyester: 5%.

The raw material, methyl 3,3-dimethyl-2-(2-methyl-1-propenyl)cyclopropanecarboxylate, was recovered in 37%.

### Example 28

Into a 100 ml flask equipped with a magnetic stirrer and a reflux condenser, 20 mg of selenium dioxide, 183 mg of pyridine and 306 mg of 42% by weight aqueous tetrafluoroboric acid solution were charged and the resulting mixture was stirred at room temperature for 10 minutes to obtain a homogeneous solution containing a selenium-containing oxidation catalyst composition. To the homogeneous solution, 1.96 g of geranyl acetate, 35 g of dichloromethane and 4.2 g of 70% by weight aqueous tert-butylhydroperoxide solution were charged and the reaction was conducted at room temperature for 24 hours. After competition of the reaction, 10 g of hexane was added to the reaction mixture and the mixture was separated to obtain an aqueous layer containing the selenium-containing oxidation catalyst composition and a hexane layer containing oxygen-containing compounds.

### <Products and Yields thereof>

E,E-2,6-dimethyl-8-acetoxy-2,6-octadien-1-ol: 15%, E,E-2-formyl-8-acetoxy-6-methyl-2,6-octadiene: 57%.

The raw material, geranyl acetate, was recovered in 1%.

### Example 29

Into a 50 ml flask equipped with a magnetic stirrer and a reflux condenser, 570 mg of selenium dioxide, 2.6 g of N-methylimidazole and 5.6 g of 42% by weight aqueous tetrafluoroboric acid solution were charged and the resulting mixture was stirred at room temperature for 10 minutes to obtain a homogeneous solution containing a selenium-containing oxidation catalyst composition. To the homogeneous solution, 8.6 g of 30% by weight aqueous hydrogen peroxide solution and 5 g of cyclohexanone were charged and the reaction was conducted at an inner temperature of 60°C for 1 hour. After competition of the reaction, 10 g of ethyl acetate was added to the reaction mixture to separate to an aqueous layer containing the selenium-containing oxidation catalyst composition and an ethyl acetate layer containing oxygen-containing compounds. The aqueous layer containing the selenium-containing oxidation catalyst composition was extracted twice with ethyl acetate to obtain an organic layer and an aqueous layer containing the selenium-containing oxidation catalyst composition. The organic layer obtained was mixed with the ethyl acetate layer obtained before to obtain an ethyl acetate layer containing cyclopentanecarboxylic acid. Yield: 62%.

### Example 30

Into a 100 ml flask equipped with a magnetic stirrer and a reflux condenser, the aqueous layer containing the selenium-containing oxidation catalyst composition obtained in Example 29, 8.6 g of 30% by weight aqueous hydrogen peroxide and 5 g of cyclohexanone were charged and the reaction was conducted at an inner temperature of 60°C for 3 hours. After competition of the reaction, post-handling was conducted as same manner as that described in Example 29, and an ethyl acetate layer containing cyclopentanecarboxylic acid. Yield: 54%. The raw material, cyclohexanone, was recovered in 15%.

### Example 31

Into a 50 ml flask equipped with a magnetic stirrer and a reflux condenser, 110 mg of selenium dioxide, 500 mg of N-methylimidazole and 1.06 g of 42% by weight aqueous tetrafluoroboric acid solution were charged and the resulting mixture was stirred at room temperature for 10 minutes to obtain a homogeneous solution containing a selenium-containing oxidation catalyst composition. To the homogeneous solution, 1.13 g of 30% by weight aqueous hydrogen peroxide solution and 1.12 g of 1,4-cyclohexanedione were charged and the reaction was conducted at an inner temperature of 60°C for 1 hour. After competition of the reaction, 10 g of ethyl acetate was added to the reaction mixture to separate to an aqueous layer containing the selenium-containing oxidation catalyst composition and an ethyl acetate layer containing oxygen-containing compounds. The aqueous layer containing the selenium-containing oxidation catalyst composition was extracted twice with ethyl acetate to obtain an organic layer and an aqueous layer containing the selenium-containing oxidation catalyst composition. The organic layer obtained was mixed with the ethyl acetate layer obtained before to obtain an ethyl acetate layer containing 3-oxocyclopentanecarboxylic acid. Yield: 44%.

### Example 32

Into a 100 ml flask equipped with a magnetic stirrer and a reflux condenser, 50 mg of selenium dioxide, 1000 mg of 1-butyl-3-methylimidazolium tetrafluoroborate, 120 mg of benzimidazole and 161 mg of benzenesulfonic acid monohydrate were charged and the resulting mixture was stirred at room temperature for 10 minutes to obtain a homogeneous solution containing a selenium compound. To the homogeneous solution, 2.5 g of 70% by weight aqueous tert-butylhydroperoxide solution and 1.5 g of methyl 3,3-dimethyl-2-(2-methyl-1-propenyl)cyclopropanecarboxylate were charged and the reaction was conducted at an inner temperature of 60°C for 6 hours. After competition of the reaction, the reaction mixture was cooled to roomtemperature and 10 g of hexane was added thereto to separate to an aqueous layer containing the selenium compound and the ionic liquid, and a hexane layer. The aqueous layer containing the selenium compound and the ionic liquid was extracted twice with hexane and the organic layer obtained was mixed with the hexane layer obtained before to obtain a hexane layer containing oxygen-containing compounds.

### <Products and Yields thereof>

Hydroxyester: 27%, formylester: 44%, carboxyester: 9%.

The raw material, methyl 3,3-dimethyl-2-(2-methyl-1-propenyl)cyclopropanecarboxylate, was recovered in 3%.

### Comparative Example 2

According to the same manner as that described in Example 32, a hexane layer containing oxygen-containing compounds was obtained except that 161 mg of benzenesulfonic acid monohydrate was not used.

### <Products and Yields thereof>

Hydroxyester: 27%, formylester: 17%, carboxyester: 4%.

The raw material, methyl 3,3-dimethyl-2-(2-methyl-1-propenyl)cyclopropanecarboxylate, was recovered in 50%.

### Industrial Applicability

According to the present invention, chrysanthemic acid derivatives which are important as acid parts of household agents for epidemic prevention and insecticides, intermediates in natural-product synthesis, and the like can be produced.

## Claims

1. An oxidation catalyst composition comprising a mixture of a selenium compound, a nitrogen-containing aromatic compound and an acid, wherein
the selenium compound is selenium dioxide or an alkali metal selenite,
the nitrogen-containing aromatic compound is pyridine, 2-methylpyridine, 3-butylpyridine, collidine, quinoline, 1,10-phenanthroline, imidazole, N-methylimidazole, 2-ethyl-4-methylimidazole or benzimidazole, and
the acid is tetrafluoroboric acid, phosphoric acid or benzenesulfonic acid.

2. The oxidation catalyst composition according to claim 1, wherein
the amount of the nitrogen-containing aromatic compound to be used is 0.5 to 10 moles relative to 1 mole of the selenium compound, and
the amount of the acid to be used is 0.5 to 2 moles relative to 1 mole of the nitrogen-containing aromatic compound.

3. An aqueous oxidation catalyst solution comprising a mixture of a selenium compound, a nitrogen-containing aromatic compound, an acid and water, wherein
the selenium compound is selenium dioxide or an alkali metal selenite,
the nitrogen-containing aromatic compound is pyridine, 2-methylpyridine, 3-butylpyridine, collidine, quinoline, 1,10-phenanthroline, imidazole, N-methylimidazole, 2-ethyl-4-methylimidazole or benzimidazole, and
the acid is tetrafluoroboric acid, phosphoric acid or benzenesulfonic acid.

4. An oxidation catalyst composition solution comprising a mixture of a selenium compound, a nitrogen-containing aromatic compound, an acid and an ionic liquid, wherein
the selenium compound is selenium dioxide or an alkali metal selenite,
the nitrogen-containing aromatic compound is pyridine, 2-methylpyridine, 3-butylpyridine, collidine, quinoline, 1,10-phenanthroline, imidazole, N-methylimidazole, 2-ethyl-4-methylimidazole or benzimidazole, and
the acid is tetrafluoroboric acid, phosphoric acid or benzenesulfonic acid.

5. The oxidation catalyst composition solution according to claim 4, wherein
the ionic liquid is a salt consisting of an organic cation and an anion, having a melting point of 100°C or less and being stable to hold liquid state until 300°C.

6. The oxidation catalyst composition solution according to claim 5, wherein
the organic cation is a substituted imidazolium cation, alkyl-substituted pyridinium cation, a quarternary ammonium cation, a quarternary phosphonium cation or a tertiary sulfonium cation and the anion species is a tetrafluoroborate anion, a halogen anion, a hexafluorophosphate anion, a bis(perfluoroalkanesulfonyl)amide anion, an alkylcarboxylate anion or an alkanesulfonate anion.

7. A method for producing an oxygen-containing compound comprising reacting an organic compound with an oxidizing agent in the presence of an oxidation catalyst composition comprising a mixture of a selenium compound, a nitrogen-containing aromatic compound and an acid, wherein
the selenium compound is selenium dioxide or an alkali metal selenite,
the nitrogen-containing aromatic compound is pyridine, 2-methylpyridine, 3-butylpyridine, collidine, quinoline, 1,10-phenanthroline, imidazole, N-methylimidazole, 2-ethyl-4-methylimidazole or benzimidazole,
the acid is tetrafluoroboric acid, phosphoric acid or benzenesulfonic acid,
the organic compound is an olefin compound having two or more hydrogen atoms on the carbon atom at α-position of a carbon-carbon double bond,
the oxidizing agent is an organic hydroperoxide compound, and
the oxygen-containing compound is at least one selected from an α-hydroxyolefin compound and an α-oxoolefin compound.

8. The method according to claim 7, wherein
the reaction is conducted in the presence of an ionic liquid.

9. The method according to claim 8, wherein
the ionic liquid is a salt consisting of an organic cation and an anion, having a melting point of 100°C or less and being stable to hold liquid state until 300°C.

10. The method according to claim 9, wherein
the organic cation is a substituted imidazolium cation, alkyl-substituted pyridinium cation, a quarternary ammonium cation, a quarternary phosphonium cation or a tertiary sulfonium cation and the anion species is a tetrafluoroborate anion, a halogen anion, a hexafluorophosphate anion, a bis(perfluoroalkanesulfonyl)amide anion, an alkylcarboxylate anion or an alkanesulfonate anion.

11. The method according to claim 7 or 8, wherein
the oxidation catalyst composition is recovered after completion of the reaction and the oxidation catalyst composition recovered is reused.

12. The method according to claim 7, wherein
the olefin compound having two or more hydrogen atoms on the carbon atom at α-position of a carbon-carbon double bond is an olefin compound represented by the formula (1): 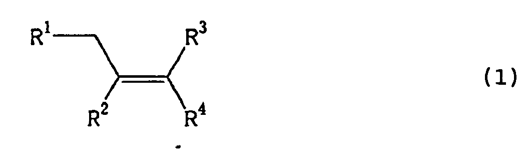 wherein R¹ represents a halogen atom; a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group which is optionally substituted with a halogen atom or atoms, a C1-C20 alkoxy group or groups, or a C6-C10 aryloxy group or groups; or a C7-C12 aralkyl group which is optionally substituted with a halogen atom or atoms, a C1-C20 alkoxy group or groups, or a C6-C10 aryloxy group or groups, and herein, the above-mentioned C1-C20 alkoxy group, C2-C10 acyl group, C6-C10 aryloxy group, C7-C12 aralkyloxy group, C2-C10 alkoxycarbonyl group and C7-C12 aryloxycarbonyl group may be substituted with a halogen atom or atoms, a C1-C20 alkoxy group or groups, or a C6-C10 aryloxy group or groups, R², R³ and R⁴ are the same or different and independently represent a hydrogen atom; a halogen atom; a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C1-C20 alkoxy group; a C2-C12 alkenyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group; a C6-C10 aryloxy group; a C7-C12 aralkyl group; a C7-C12 aralkyloxy group; a C2-C10 acyl group; a C2-C10 alkoxycarbonyl group; a C7-C12 aryloxycarbonyl group; a C8-C12 aralkyloxycarbonyl group; or a carboxyl group, and herein, the above-mentioned C1-C20 alkoxy group, C2-C10 acyl group, C6-C10 aryl group, C6-C10 aryloxy group, C7-C12 aralkyl group, C7-C12 aralkyloxy group, C2-C10 alkoxycarbonyl group, C7-C12 aryloxycarbonyl group and C8-C12 aralkyloxycarbonyl group may be substituted with a halogen atom or atoms, a C1-C20 alkoxy group or groups, or a C6-C10 aryloxy group or groups, and at least one pair selected from R¹ and R³, R² and R⁴, R¹ and R², and R³ and R⁴ may be bonded to form a ring,
the α-hydroxyolefin compound is an alcohol compound represented by the formula (2): 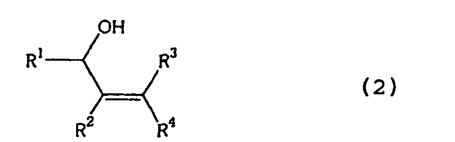 wherein R¹, R², R³ and R⁴ are the same as the described above, and
the α-oxoolefin compound is a ketone compound represented by the formula (3): 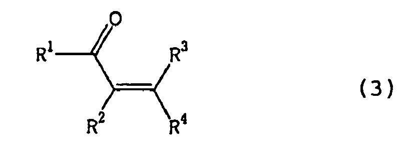 wherein R¹, R², R³ and R⁴ are the same as the described above.

13. The method according to claim 7, wherein
the olefin compound having two or more hydrogen atoms on the carbon atom at α-position of a carbon-carbon double bond is an olefin compound represented by the formula (4): 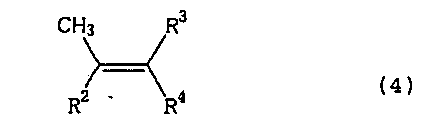 wherein R², R³ and R⁴ respectively represent the same as described above, and herein, R² and R⁴ or R³ and R⁴ may be bonded to form a ring,
the α-hydroxyolefin compound is an alcohol compound represented by the formula (5): 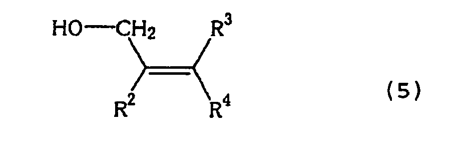 wherein R², R³ and R⁴ are the same as the described above,
the α-oxoolefin compound is at least one selected from an aldehyde compound represented by the formula (6): 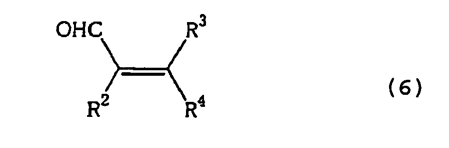 wherein R², R³ and R⁴ are the same as the described above, and a carboxylic acid compound represented by the formula (7): 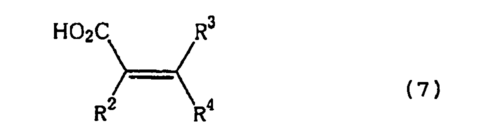 wherein R², R³ and R⁴ are the same as the described above.

14. The method according to claim 13, wherein
the olefin compound represented by the formula (4) is a chrysanthemic acid compound represented by the formula (8): 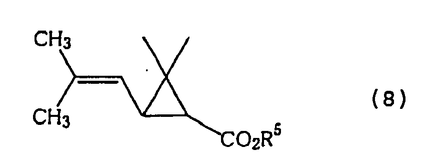 wherein R⁵ represents a C1-C20 alkyl group which is optionally substituted with a halogen atom or atoms, a C1-C20 alkoxy group or groups, a C2-C10 acyl group or groups, a C6-C10 aryloxy group or groups, a C7-C12 aralkyloxy group or groups, a C2-C10 alkoxycarbonyl group or groups, a C7-C12 aryloxycarbonyl group or groups, or a carboxyl group or groups; a C6-C10 aryl group; a C7-C12 aralkyl group or a hydrogen atom, and herein, the above-mentioned C1-C20 alkoxy group, C6-C10 aryloxy group, C7-C12 aralkyloxy group, C2-C10 alkoxycarbonyl group, C7-C12 aryloxycarbonyl group and C7-C12 aralkyl group may be substituted with a halogen atom or atoms, a C1-C20 alkoxy group or groups, or a C6-C10 aryloxy group or groups,
the alcohol compound represented by the formula (5) is an alcohol compound represented by the formula (9): 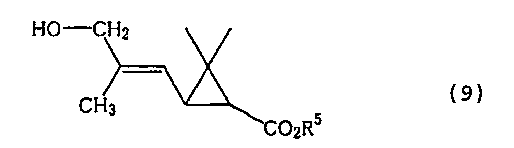 wherein R⁵ is the same as the described above,
the aldehyde compound represented by the formula (6) is an aldehyde compound represented by the formula (10): 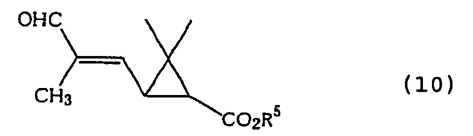 wherein R⁵ is the same as the described above, and
the carboxylic acid compound represented by the formula (7) is a carboxylic acid compound represented by the formula (11) : 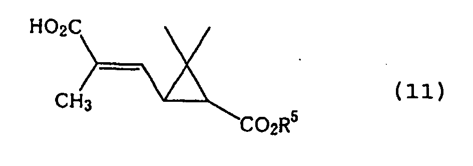 wherein R⁵ is the same as the described above.

15. The method according to claim 13, wherein
the amount of the organic hydroperoxide compound to be used is 2 to 3.5 moles relative to 1 mole of the olefin compound represented by the formula (4), and
a main component is the aldehyde compound represented by the formula (6).

16. The method according to claim 14, wherein
the amount of the organic hydroperoxide compound to be used is 2 to 3.5 moles relative to 1 mole of the chrysanthemic acid compound represented by the formula (8), and
a main component is the aldehyde compound represented by the formula (10).

17. A method for producing an oxygen-containing compound comprising reacting an organic compound with an oxidizing agent in the presence of an oxidation catalyst composition comprising a mixture of a selenium compound, a nitrogen-containing aromatic compound and an acid, wherein
the selenium compound is selenium dioxide or an alkali metal selenite,
the nitrogen-containing aromatic compound is pyridine, 2-methylpyridine, 3-butylpyridine, collidine, quinoline, 1,10-phenanthroline, imidazole, N-methylimidazole, 2-ethyl-4-methylimidazole or benzimidazole,
the acid is tetrafluoroboric acid, phosphoric acid or benzenesulfonic acid,
the organic compound is a cycloalkanone compound,
the oxidizing agent is hydrogen peroxide, and
the oxygen-containing compound is cycloalkanecarboxylic acid.

18. The method according to claim 17, wherein
the cycloalkanone compound is a cycloalkanone compound represented by the formula (12): 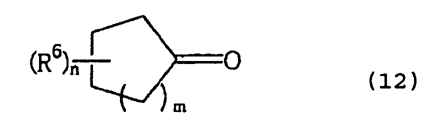 wherein R⁶ represents a hydrogen atom; a halogen atom; a C1-C20 alkyl group; a C6-C10 aryl group; a C7-C12 aralkyl group; or a C1-C20 alkoxy group, and herein, the above-mentioned C1-C20 alkyl group, C6-C10 aryl group, C7-C12 aralkyl group and C1-C20 alkoxy group may be substituted with a halogen atom or atoms, or a C1-C20 alkoxy group or groups, m represents an integer of 0 to 8, n represents an integer of 0 to 11 which satisfies n ≤ m+3, and herein, when n is 2 more, R⁶ may be the same or different, and any of two R⁶ may be bonded to form a ring, and
the cycloalkanecarboxylic acid compound is a cycloalkanecarboxylic acid compound represented by the formula (13): 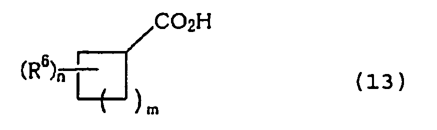 wherein R⁶, m and n are the same as the described above.

## Patentansprüche

1. Oxidationskatalysatorzusammensetzung, die ein Gemisch von einer Selenverbindung, einer stickstoffhaltigen aromatischen Verbindung und einer Säure umfasst, wobei
die Selenverbindung Selendioxid oder ein Alkalimetallselenit ist,
die stickstoffhaltige aromatische Verbindung Pyridin, 2-Methylpyridin, 3-Butylpyridin, Collidin, Chinolin, 1,10-Phenanthrolin, Imidazol, N-Methylimidazol, 2-Ethyl-4-methylimidazol oder Benzimidazol ist und
die Säure Tetrafluoroborsäure, Phosphorsäure oder Benzolsulfonsäure ist.

2. Oxidationskatalysatorzusammensetzung nach Anspruch 1, wobei
die zu verwendende Menge der stickstoffhaltigen aromatischen Verbindung 0,5 bis 10 mol, bezogen auf 1 mol der Selenverbindung, beträgt und
die zu verwendende Menge der Säure 0,5 bis 2 mol, bezogen auf 1 mol der stickstoffhaltigen aromatischen Verbindung, beträgt.

3. Wässrige Oxidationskatalysatorlösung, die ein Gemisch von einer Selenverbindung, einer stickstoffhaltigen aromatischen Verbindung, einer Säure und Wasser umfasst, wobei
die Selenverbindung Selendioxid oder ein Alkalimetallselenit ist,
die stickstoffhaltige aromatische Verbindung Pyridin, 2-Methylpyridin, 3-Butylpyridin, Collidin, Chinolin, 1,10-Phenanthrolin, Imidazol, N-Methylimidazol, 2-Ethyl-4-methylimidazol oder Benzimidazol ist und
die Säure Tetrafluoroborsäure, Phosphorsäure oder Benzolsulfonsäure ist.

4. Oxidationskatalysatorzusammensetzungslösung, die ein Gemisch von einer Selenverbindung, einer stickstoffhaltigen aromatischen Verbindung, einer Säure und einer ionischen Flüssigkeit umfasst, wobei
die Selenverbindung Selendioxid oder ein Alkalimetallselenit ist,
die stickstoffhaltige aromatische Verbindung Pyridin, 2-Methylpyridin, 3-Butylpyridin, Collidin, Chinolin, 1,10-Phenanthrolin, Imidazol, N-Methylimidazol, 2-Ethyl-4-methylimidazol oder Benzimidazol ist und
die Säure Tetrafluoroborsäure, Phosphorsäure oder Benzolsulfonsäure ist.

5. Oxidationskatalysatorzusammensetzungslösung nach Anspruch 4, wobei
die ionische Flüssigkeit ein Salz ist, das aus einem organischen Kation und einem Anion besteht, einen Schmelzpunkt von 100 °C oder weniger aufweist und so stabil ist, dass es den flüssigen Zustand bis 300 °C beibehält.

6. Oxidationskatalysatorzusammensetzungslösung nach Anspruch 5, wobei
das organische Kation ein substituiertes Imidazoliumkation, ein alkylsubstituiertes Pyridiniumkation, ein quaternäres Ammoniumkation, ein quaternäres Phosphoniumkation oder ein tertiäres Sulfoniumkation ist und die Anionspezies ein Tetrafluoroboratanion, ein Halogenanion, ein Hexafluorophosphatanion, ein Bis(perfluoralkansulfonyl)amidanion, ein Alkylcarboxylatanion oder ein Alkansulfonatanion ist.

7. Verfahren zur Herstellung einer sauerstoffhaltigen Verbindung, umfassend das Umsetzen einer organischen Verbindung mit einem Oxidationsmittel in Gegenwart einer Oxidationskatalysatorzusammensetzung, die ein Gemisch von einer Selenverbindung, einer stickstoffhaltigen aromatischen Verbindung und einer Säure umfasst, wobei
die Selenverbindung Selendioxid oder ein Alkalimetallselenit ist,
die stickstoffhaltige aromatische Verbindung Pyridin, 2-Methylpyridin, 3-Butylpyridin, Collidin, Chinolin, 1,10-Phenanthrolin, Imidazol, N-Methylimidazol, 2-Ethyl-4-methylimidazol oder Benzimidazol ist und
die Säure Tetrafluoroborsäure, Phosphorsäure oder Benzolsulfonsäure ist,
die organische Verbindung eine Olfinverbindung mit zwei oder mehr Wasserstoffatomen an dem Kohlenstoffatom in α-Position zu einer Kohlenstoff-Kohlenstoff-Doppelbindung ist,
das Oxidationsmittel eine organische Hydroperoxidverbindung ist und
die sauerstoffhaltige Verbindung mindestens eine Verbindung ist, die aus einer α-Hydroxyolefinverbindung und einer α-Oxoolefinverbindung ausgewählt ist.

8. Verfahren nach Anspruch 7, wobei die Reaktion in Gegenwart einer ionischen Flüssigkeit durchgeführt wird.

9. Verfahren nach Anspruch 8, wobei
die ionische Flüssigkeit ein Salz ist, das aus einem organischen Kation und einem Anion besteht, einen Schmelzpunkt von 100 °C oder weniger aufweist und so stabil ist, dass es den flüssigen Zustand bis 300 °C beibehält.

10. Verfahren nach Anspruch 9, wobei
das organische Kation ein substituiertes Imidazoliumkation, ein alkylsubstituiertes Pyridiniumkation, ein quaternäres Ammoniumkation, ein quaternäres Phosphoniumkation oder ein tertiäres Sulfoniumkation ist und die Anionspezies ein Tetrafluoroboratanion, ein Halogenanion, ein Hexafluorophosphatanion, ein Bis(perfluoralkansulfonyl)amidanion, ein Alkylcarboxylatanion oder ein Alkansulfonatanion ist.

11. Verfahren nach Anspruch 7 oder 8, wobei die Oxidationskatalysatorzusammensetzung nach der Durchführung der Reaktion zurückgewonnen wird und die zurückgewonnene Oxidationskatalysatorzusammensetzung wiederverwendet wird.

12. Verfahren nach Anspruch 7, wobei
die Olefinverbindung mit zwei oder mehr Wasserstoffatomen an den Kohlenstoffatomen in α-Position zu einer Kohlenstoff-Kohlenstoff-Doppelbindung eine Olefinverbindung der Formel (1) ist: 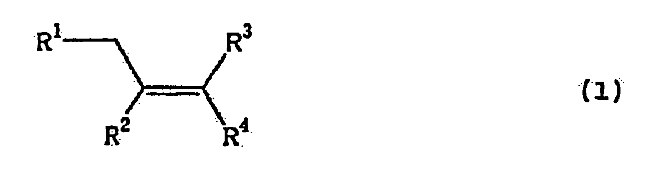 worin R¹ für ein Halogenatom; eine C₁-C₂₀-Alkylgruppe, die optional mit einem oder mehreren Halogenatomen, einer oder mehreren C₁-C₂₀-Alkoxygruppen, einer oder mehreren C₂-C₁₀-Acylgruppen, einer oder mehreren C₆-C₁₀-Aryloxygruppen, einer oder mehreren C₇-C₁₂-Aralkyloxygruppen, einer oder mehreren C₂-C₁₀-Alkoxycarbonylgruppen, einer oder mehreren C₇-C₁₂-Aryloxycarbonylgruppen oder einer oder mehreren Carboxylgruppen substituiert ist; eine C₆-C₁₀-Arylgruppe, die optional mit einem oder mehreren Halogenatomen, einer oder mehreren C₁-C₂₀-Alkoxygruppen oder einer oder mehreren C₆-C₁₀-Aryloxygruppen substituiert ist; oder eine C₇-C₁₂-Aralkylgruppe, die optional mit einem oder mehreren Halogenatomen, einer oder mehreren C₁-C₂₀-Alkoxygruppen oder einer oder mehreren C₆-C₁₀-Aryloxygruppen substituiert ist, steht, und hierbei die im vorhergehenden genannte C₁-C₂₀-Alkoxygruppe, C₂-C₁₀-Acylgruppe, C₆-C₁₀-Aryloxygruppe, C₇-C₁₂-Aralkyloxygruppe, C₂-C₁₀-Alkoxycarbonylgruppe und C₇-C₁₂-Aryloxycarbonylgruppe mit einem oder mehreren Halogenatomen, einer oder mehreren C₁-C₂₀-Alkoxygruppen oder einer oder mehreren C₆-C₁₀-Aryloxygruppen substituiert sein können, R², R³ und R⁴ gleich oder verschieden sind und unabhängig voneinander für ein Wasserstoffatom; ein Halogenatom; eine C₁-C₂₀-Alkylgruppe, die optional mit einem oder mehreren Halogenatomen, einer oder mehreren C₁-C₂₀-Alkoxygruppen, einer oder mehreren C₂-C₁₀-Acylgruppen, einer oder mehreren C₆-C₁₀-Aryloxygruppen, einer oder mehreren C₇-C₁₂-Aralkyloxygruppen, einer oder mehreren C₂-C₁₀-Alkoxycarbonylgruppen, einer oder mehreren C₇-C₁₂-Aryloxycarbonylgruppen oder einer oder mehreren Carboxylgruppen substituiert ist;
eine C₁-C₂₀-Alkoxygruppe; eine C₂-C₁₂-Alkenylgruppe, die optional mit einem oder mehreren Halogenatomen, einer oder mehreren C₁-C₂₀-Alkoxygruppen, einer oder mehreren C₂-C₁₀-Acylgruppen, einer oder mehreren C₆-C₁₀-Aryloxygruppen, einer oder mehreren C₇-C₁₂-Aralkyloxygruppen, einer oder mehreren C₂-C₁₀-Alkoxycarbonylgruppen, einer oder mehreren C₇-C₁₂-Aryloxycarbonylgruppen oder einer oder mehreren Carboxylgruppen substituiert ist; eine C₆-C₁₀-Arylgruppe; eine C₆-C₁₀-Aryloxygruppe; eine C₇-C₁₂-Aralkylgruppe; eine C₇-C₁₂-Aralkyloxygruppe; eine C₂-C₁₀-Acylgruppe; eine C₂-C₁₀-Alkoxycarbonylgruppe; eine C₇-C₁₂-Aryloxycarbonylgruppe; eine C₈-C₁₂-Aralkyloxycarbonylgruppe oder eine Carboxylgruppe stehen und hierbei die im vorhergehenden genannte C₁-C₂₀-Alkoxygruppe, C₂-C₁₀-Acylgruppe, C₆-C₁₀-Arylgruppe, C₆-C₁₀-Aryloxygruppe, C₇-C₁₂-Aralkylgruppe, C₇-C₁₂-Aralkyloxygruppe, C₂-C₁₀-Alkoxycarbonylgruppe, C₇-C₁₂-Aryloxycarbonylgruppe und C₈-C₁₂-Aralkyloxycarbonylgruppe mit einem oder mehreren Halogenatomen, einer oder mehreren C₁-C₂₀-Alkoxygruppen oder einer oder mehreren C₆-C₁₀-Aryloxygruppen substituiert sein können und mindestens ein Paar, das aus R¹ und R³, R² und R⁴, R¹ und R² und R³ und R⁴ ausgewählt ist, unter Bildung eines Rings verbunden sein kann,
die α-Hydroxyolefinverbindung eine Alkoholverbindung der Formel (2) ist: 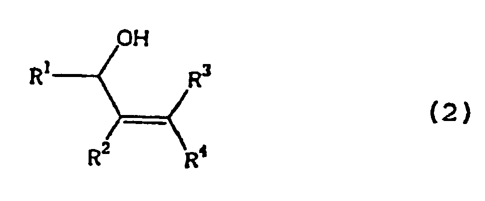 worin R¹, R² und R³ und R⁴ gleich den im vorhergehenden beschriebenen sind, und
die α-Oxoolefinverbindung eine Ketonverbindung der Formel (3) ist: 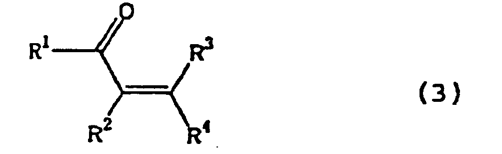 worin R¹, R², R³ und R⁴ gleich den im vorhergehenden beschriebenen sind.

13. Verfahren nach Anspruch 7, wobei
die Olefinverbindung mit zwei oder mehr Wasserstoffatomen an dem Kohlenstoffatom in der α-position zu einer Kohlenstoff-Kohlenstoff-Doppelbindung eine Olefinverbindung der Formel (4) ist: 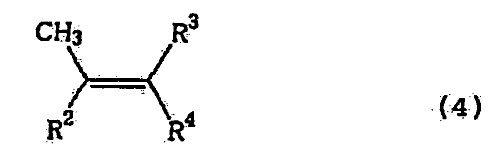 worin R², R³ und R⁹ jeweils gleich den im vorhergehenden beschriebenen sind und hierbei R² und R⁴ oder R³ und R⁴ unter Bildung eines Rings verbunden sein können,
die α-Hydroxyolefinverbindung eine Alkoholverbindung der Formel (5) ist: 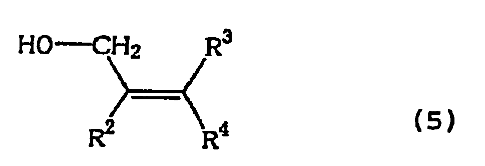 worin R², R³ und R⁴ gleich den im vorhergehenden beschriebenen sind,
die α-Oxoolefinverbindung mindestens eine Verbindung ist, die ausgewählt ist aus einer Aldehydverbindung der Formel (6): 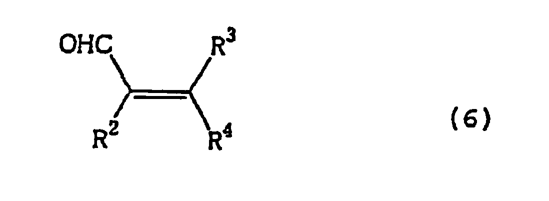 worin R², R³ und R⁴ gleich den im vorhergehenden beschriebenen sind,
und einer Carbonsäureverbindung der Formel (7): 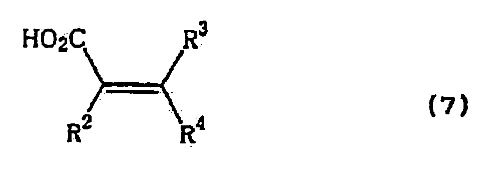 worin R², R³ und R⁴ gleich den im vorhergehenden beschriebenen sind.

14. Verfahren nach Anspruch 13, wobei
die Olefinverbindung der Formel (4) eine Chrysanthemsäureverbindung der Formel (8) ist: 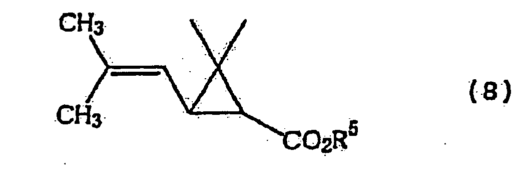 worin R⁵ für eine C₁-C₂₀-Alkylgruppe, die optional mit einem oder mehreren Halogenatomen, einer oder mehreren C₁-C₂₀-Alkoxygruppen, einer oder mehreren C₂-C₁₀-Acylgruppen, einer oder mehreren C₆-C₁₀-Aryloxygruppen, einer oder mehreren C₇-C₁₂-Aralkyloxygruppen, einer oder mehreren C₂-C₁₀-Alkoxycarbonylgruppen, einer oder mehreren C₇-C₁₂-Aryloxycarbonylgruppen oder einer oder mehreren Carboxylgruppen substituiert ist; eine C₆-C₁₀-Arylgruppe; eine C₇-C₁₂-Aralkylgruppe oder ein Wasserstoffatom steht und hierbei die im vorhergehenden genannte C₁-C₂₀-Alkoxygruppe, C₆-C₁₀-Aryloxygruppe, C₇-C₁₂-Aralkyloxygruppe, C₂-C₁₀-Alkoxycarbonylgruppe, C₇-C₁₂-Aryloxycarbonylgruppe und C₇-C₁₂-Aralkylgruppe mit einem oder mehreren Halogenatomen, einer oder mehreren C₁-C₂₀-Alkoxygruppen oder einer oder mehreren C₆-C₁₀-Aryloxygruppen substituiert sein können,
die Alkoholverbindung der Formel (5) eine Alkoholverbindung der Formel (9) ist: 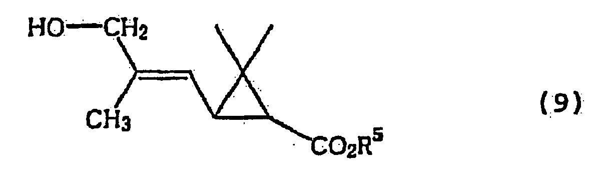 worin R⁵ gleich dem im vorhergehenden beschriebenen ist,
die Aldehydverbindung der Formel (6) eine Aldehydverbindung der Formel (10) ist: 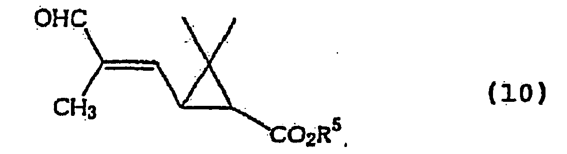 worin R⁵ gleich dem im vorhergehenden beschriebenen ist, und
die Carbonsäureverbindung der Formel (7) eine Carbonsäureverbindung der Formel (11) ist: 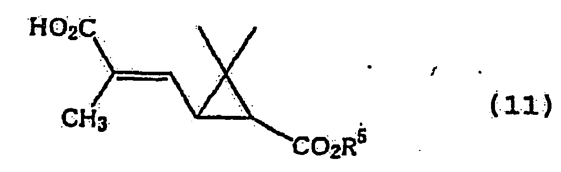 worin R⁵ gleich dem im vorhergehenden beschriebenen ist.

15. Verfahren nach Anspruch 13, wobei
die zu verwendende Menge der organischen Hydroperoxidverbindung 2 bis 3,5 mol, bezogen auf 1 mol der Olefinverbindung der Formel (4), beträgt und
eine Hauptkomponente die Aldehydverbindung der Formel (6) ist.

16. Verfahren nach Anspruch 14, wobei
die zu verwendende Menge der organischen Hydroperoxidverbindung 2 bis 3,5 mol, bezogen auf 1 mol der Chrysanthemsäureverbindung der Formel (8), beträgt und
eine Hauptkomponente die Aldehydverbindung der Formel (10) ist.

17. Verfahren zur Herstellung einer sauerstoffhaltigen Verbindung, umfassend das Umsetzen einer organischen Verbindung mit einem Oxidationsmittel in Gegenwart einer Oxidationskatalysatorzusammensetzung, die ein Gemisch von einer Selenverbindung, einer stickstoffhaltigen aromatischen Verbindung und einer Säure umfasst,
wobei die Selenverbindung Selendioxid oder ein Alkalimetallselenit ist,
die stickstoffhaltige aromatische Verbindung Pyridin, 2-Methylpyridin, 3-Butylpyridin, Collidin, Chinolin, 1,10-Phenanthrolin, Imidazol, N-Methylimidazol, 2-Ethyl-4-methylimidazol oder Benzimidazol ist,
die Säure Tetrafluoroborsäure, Phosphorsäure oder Benzolsulfonsäure ist,
die organische Verbindung eine Cycloalkanonverbindung ist,
das Oxidationsmittel Wasserstoffperoxid ist und
die sauerstoffhaltige Verbindung eine Cycloalkancarbonsäure ist.

18. Verfahren nach Anspruch 17, wobei
die Cycloalkanonverbindung eine Cycloalkanonverbindung der Formel (12) ist: 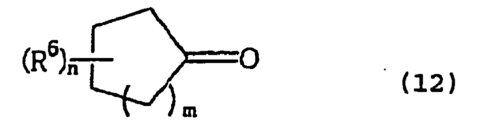 worin R⁶ für ein Wasserstoffatom; ein Halogenatom; eine C₁-C₂₀-Alkylgruppe; eine C₆-C₁₀-Arylgruppe; eine C₇-C₁₂-Aralkylgruppe; oder eine C₁-C₂₀-Alkoxygruppe steht und hierbei die im vorhergehenden genannte C₁-C₂₀-Alkylgruppe, C₆-C₁₀-Arylgruppe, C₇-C₁₂-Aralkylgruppe und C₁-C₂₀-Alkoxygruppe mit einem oder mehreren Halogenatomen oder einer oder mehreren C₁-C₂₀-Alkoxygruppen substituiert sein können, m für eine ganze Zahl von 0 bis 8 steht, n für eine ganze Zahl von 0 bis 11 steht, wobei n ≤ m+3 erfüllt ist, und hierbei, wenn n 2 oder mehr beträgt, die Reste R⁶ gleich oder verschieden sein können und beliebige zwei Reste R⁶ unter Bildung eines Rings verbunden sein können, und
die Cycloalkancarbonsäureverbindung eine Cycloalkancarbonsäureverbindung der Formel (13) ist: 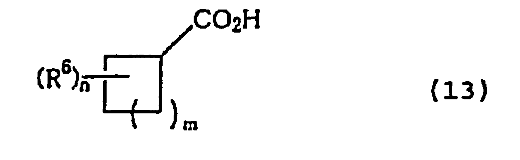 worin R⁶, m und n gleich den im vorhergehenden beschriebenen sind.

## Revendications

1. Composition de catalyseur d'oxydation comprenant un mélange d'un composé de sélénium, d'un composé aromatique contenant de l'azote et d'un acide, dans laquelle
le composé de sélénium est le dioxyde de sélénium ou un séléniure de métal alcalin,
le composé aromatique contenant de l'azote est la pyridine, la 2-méthylpyridine, la 3-butylpyridine, la collidine, la quinoléine, la 1,10-phénanthroline, l'imidazole, le N-méthylimidazole, le 2-éthyl-4-méthylimidazole ou le benzimidazole, et
l'acide est l'acide tétrafluoroborique, l'acide phosphorique ou l'acide benzènesulfonique.

2. Composition de catalyseur d'oxydation selon la revendication 1, dans laquelle
la quantité du composé aromatique contenant de l'azote à utiliser est de 0,5 à 10 mol par rapport à 1 mol du composé de sélénium, et
la quantité de l'acide à utiliser est de 0,5 à 2 mol par rapport à 1 mol du composé aromatique contenant de l'azote.

3. Solution aqueuse de catalyseur d'oxydation comprenant un mélange d'un composé de sélénium, d'un composé aromatique contenant de l'azote, d'un acide et d'eau, dans laquelle
le composé de sélénium est le dioxyde de sélénium ou un séléniure de métal alcalin,
le composé aromatique contenant de l'azote est la pyridine, la 2-méthylpyridine, la 3-butylpyridine, la collidine, la quinoléine, la 1,10-phénanthroline, l'imidazole, le N-méthylimidazole, le 2-éthyl-4-méthylimidazole ou le benzimidazole, et
l'acide est l'acide tétrafluoroborique, l'acide phosphorique ou l'acide benzènesulfonique.

4. Solution de composition de catalyseur d'oxydation comprenant un mélange d'un composé de sélénium, d'un composé aromatique contenant de l'azote, d'un acide et d'un liquide ionique, dans laquelle
le composé de sélénium est le dioxyde de sélénium ou un séléniure de métal alcalin,
le composé aromatique contenant de l'azote est la pyridine, la 2-méthylpyridine, la 3-butylpyridine, la collidine, la quinoléine, la 1,10-phénanthroline, l'imidazole, le N-méthylimidazole, le 2-éthyl-4-méthylimidazole ou le benzimidazole, et
l'acide est l'acide tétraflurooborique, l'acide phosphorique ou l'acide benzènesulfonique.

5. Solution de composition de catalyseur d'oxydation selon la revendication 4, dans laquelle
le liquide ionique est un sel constitué d'un cation organique et d'un anion, ayant un point de fusion de 100°C ou inférieur et étant stable pour maintenir l'état liquide jusqu'à 300°C.

6. Solution de composition de catalyseur d'oxydation selon la revendication 5, dans laquelle
le cation organique est un cation d'imidazolium substitué, un cation de pyridinium alkyle-substitué, un cation d'ammonium quaternaire, un cation de phosphonium quaternaire ou un cation de sulfonium tertiaire et le type d'anion est un anion de tétrafluoroborate, un anion d'halogène, un anion d'hexafluorophosphate, un anion de bis(perfluoroalcanesulfonyl) amide et un anion de carboxylate d'alkyle ou un anion de sulfonate d'alcane.

7. Procédé de production d'un composé contenant de l'oxygène comprenant la réaction d'un composé organique avec un agent oxydant en présence d'une composition de catalyseur d'oxydation comprenant un mélange d'un composé de sélénium, d'un composé aromatique contenant de l'azote et d'un acide, dans lequel
le composé de sélénium est le dioxyde de sélénium ou un séléniure de métal alcalin,
le composé aromatique contenant de l'azote est la pyridine, la la 2-méthylpyridine, la 3-butylpyridine, la collidine, la quinoléine, la 1,10-phénanthroline, l'imidazole, le N-méthylimidazole, le 2-éthyl-4-méthylimidazole ou le benzimidazole,
l'acide est l'acide tétrafluoroborique, l'acide phosphorique ou l'acide benzènesulfonique,
le composé organique est un composé d'oléfine ayant 2 ou plusieurs atomes d'hydrogène sur l'atome de carbone à la position α d'une double liaison carbone-carbone,
l'agent oxydant est un composé d'hydroperoxyde organique, et
le composé contenant de l'oxygène est au moins un choisi parmi un composé d'hydroxyoléfine α et un composé d'oxooléfine α.

8. Procédé selon la revendication 7, dans lequel
la réaction est réalisée en présence d'un liquide ionique.

9. Procédé selon la revendication 8, dans lequel
le liquide ionique est un sel constitué d'un cation organique et d'un anion, ayant un point de fusion de 100°C ou inférieur et étant stable pour maintenir l'état liquide jusqu'à 300°C.

10. Procédé selon la revendication 9, dans lequel
le cation organique est un cation d'imidazolium substitué, un cation de pyridinium alkyle-substitué, un cation d'ammonium quaternaire, un cation de phosphonium quaternaire ou un cation de sulfonium tertiaire et le type d'anion est un anion de tétrafluoroborate, un anion d'halogène, un anion d'hexafluorophosphate, un anion de bis(perfluoroalcanesulfonyl) amide, un anion de carboxylate d'alkyle et un anion de sulfonate d'alcane.

11. Procédé selon la revendication 7 ou 8, dans lequel la composition de catalyseur d'oxydation est récupérée après l'achèvement de la réaction et la composition de catalyseur d'oxydation récupérée est réutilisée.

12. Procédé selon la revendication 7, dans lequel
le composé d'oléfine présentant deux ou plusieurs atomes d'hydrogène sur l'atome de carbone à la position α d'une double liaison carbone-carbone est un composé d'oléfine représenté par la formule (1) : 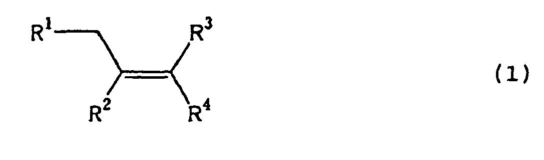 dans laquelle R¹ représente un atome d'halogène ; un groupe alkyle en C1-C20 qui est éventuellement substitué avec un atome ou des atomes d'halogène, un groupe ou des groupes alcoxy en C1-C20, un groupe ou des groupes acyle en C2-C10, un groupe ou des groupes aryloxy en C6-C10, un groupe ou des groupes aralkyloxy en C7-C12, un groupe ou des groupes alcoxycarbonyle en C2-C10, un groupe ou des groupes aryloxycarbonyle en C7-C12 ou un groupe ou des groupes carboxyle ; un groupe aryle en C6-C10 qui est éventuellement substitué avec un atome ou des atomes d'halogène, un groupe ou des groupes alcoxy en C1-C20 ou un groupe ou des groupes aryloxy en C6-C10 ; ou un groupe aralkyle en C7-C12 qui est éventuellement substitué avec un atome ou des atomes d'halogène, un groupe ou des groupes alcoxy en C1-C20 ou un groupe ou des groupes aryloxy en C6-C10, et dans lequel les groupes alcoxy en C1-C20, groupe acyle en C2-C10, groupe aryloxy en C6-C10, groupe aralkyloxy en C7-C12, groupe alcoxycarbonyle en C2-C10 et groupe aryloxycarbonyle en C7-C10 cités ci-dessus peuvent être substitués avec un atome ou des atomes d'halogène, un groupe ou des groupes alcoxy en C1-C20 ou un groupe ou des groupes aryloxy en C6-C10, R², R³ et R⁴ sont identiques ou différents et représentent indépendamment un atome d'hydrogène ; un atome d'halogène ; un groupe alkyle en C1-C20 qui est éventuellement substitué avec un atome ou des atomes d'halogène, un groupe ou des groupes alcoxy en C1-C20, un groupe ou des groupes acyle en C2-C10, un groupe ou des groupes aryloxy en C6-C10, un groupe ou des groupes aralkyloxy en C7-C12, un groupe ou des groupes alcoxycarbonyle en C2-C10, un groupe ou des groupes aryloxycarbonyle en C7-C12, ou un groupe ou des groupes carboxyle ; un groupe alcoxy en C1-C20 ; un groupe alcényle en C2-C12 qui est éventuellement substitué avec un atome ou des atomes d'halogène, un groupe ou des groupes alcoxy en C1-C20, un groupe ou des groupes acyle en C2-C10, un groupe ou des groupes aryloxy en C6-C10, un groupe ou des groupes aralkyloxy en C7-C12, un groupe ou des groupes alcoxycarbonyle en C2-C10, un groupe ou des groupes aryloxycarbonyle en C7-C12 ou un groupe ou des groupes carboxyle ; un groupe aryle en C6-C10 ; un groupe aryloxy en C6-C10 ; un groupe aralkyle en C7-C12 ; un groupe aralkyloxy en C7-C12 ; un groupe acyle en C2-C10 ; un groupe alcoxycarbonyle en C2-C10 ; un groupe aryloxycarbonyle en C7-C12 ; un groupe aralkyloxycarbonyle en C8-C12 ; ou un groupe carboxyle, et dans lequel les groupe alcoxy en C1-C20, groupe acyle en C2-C10, groupe aryle en C6-C10, groupe aryloxy en C6-C10, groupe aralkyle en C7-C12, groupe aralkyloxy en C7-C12, groupe alcoxycarbonyle en C2-C10, groupe aryloxycarbonyle en C7-C12 et groupe aralkyloxycarbonyle en C8-C12 cités ci-dessus peuvent être substitués avec un atome ou des atomes d'halogène, un groupe ou des groupes alcoxy en C1-C20 ou un groupe ou des groupes aryloxy en C6-C10, et au moins une paire choisie parmi R¹ et R³, R² et R⁴, R¹ et R², et R³ et R⁴ peut être liée pour former un noyau,
le composé d'hydroxyoléfine α est un composé d'alcool représenté par la formule (2) : 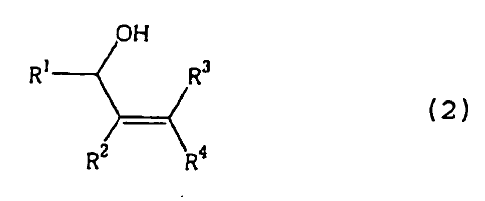 dans laquelle R¹, R², R³ et R⁴ sont identiques à ceux décrits ci-dessus, et
le composé d'oxooléfine α est un composé de cétone représenté par la formule (3) : 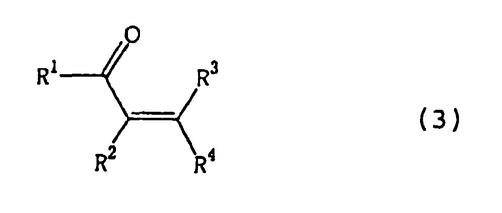 dans laquelle R¹, R², R³ et R⁴ sont identiques à ceux décrits ci-dessous.

13. Procédé selon la revendication 7, dans lequel
le composé d'oléfine présentant deux ou plusieurs atomes d'hydrogène sur l'atome de carbone à la position α d'une double liaison carbone-carbone est un composé d'oléfine représenté par la formule (4) : 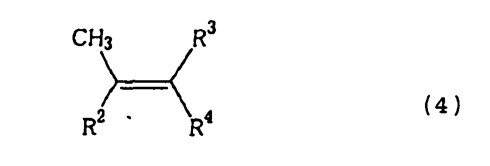 dans laquelle R², R³ et R⁴ représentent respectivement ceux comme décrit ci-dessus et R² et R⁴ ou R³ et R⁴ peuvent ici être liés pour former un noyau,
le composé d'hydroxyoléfine α est un composé d'alcool représenté par la formule (5) : 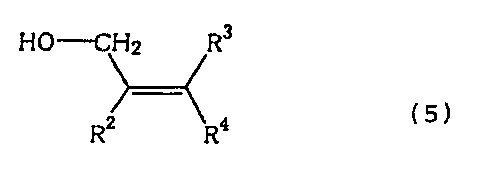 dans laquelle R², R³ et R⁴ sont identiques à ceux décrits ci-dessus,
le composé d'oxooléfine α est au moins un choisi parmi un composé d'aldéhyde représenté par la formule (6) : 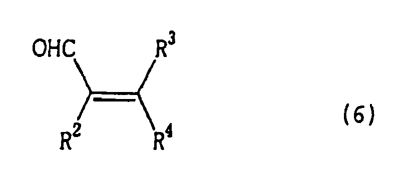 dans laquelle R², R³ et R⁴ sont identiques à ceux décrits ci-dessus, et un composé d'acide carboxylique représenté par la formule (7) : 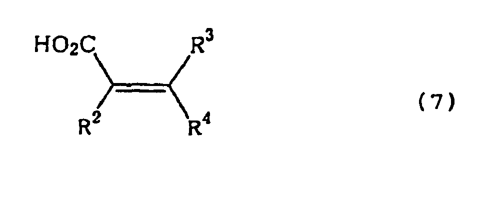 dans laquelle R², R³ et R⁴ sont identiques à ceux décrits ci-dessus.

14. Procédé selon la revendication 13, dans lequel
le composé d'oléfine représenté par la formule (4) est un composé d'acide chrysanthémique représenté par la formule (8) : 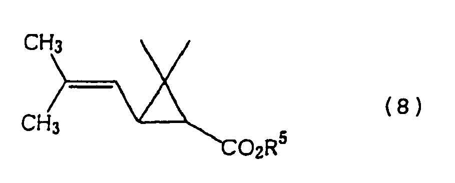 dans laquelle R⁵ représente un groupe alkyle en C1-C20 qui est éventuellement substitué avec un atome ou des atomes d'halogène, un groupe ou des groupes alcoxy en C1-C20, un groupe ou des groupes acyle en C2-C10, un groupe ou des groupes aryloxy en C6-C10, un groupe ou des groupes aralkyloxy en C7-C12, un groupe ou des groupes alcoxycarbonyle en C2-C10, un groupe ou des groupes aryloxycarbonyle en C7-C12, ou un groupe ou des groupes carboxyle ; un groupe aryle en C6-C10 ; un groupe aralkyle en C7-C12 ou un atome d'hydrogène, et les groupe alcoxy C1-C20, groupe aryloxy en C6-C10, groupe aralkyloxy en C7-C12, groupe alcoxycarbonyle en C2-C10, groupe aryloxycarbonyle en C7-C12 et groupe aralkyle en C7-C12 cités ci-dessus peuvent ici être substitués avec un atome ou des atomes d'halogène, un groupe ou des groupes alcoxy en C1-C20 ou un groupe ou des groupes aryloxy en C6-C10,
le composé d'alcool représenté par la formule (5) est un composé d'alcool représenté par la formule (9) : 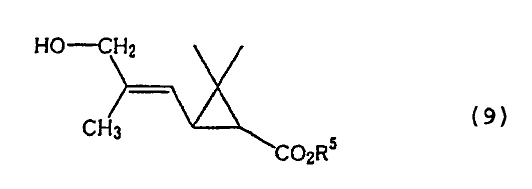 dans laquelle R⁵ est identique à celui décrit ci-dessus,
le composé d'aldéhyde représenté par la formule (6) est un composé d'aldéhyde représenté par la formule (10) : 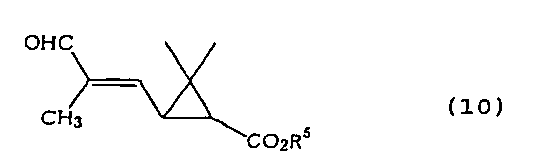 dans laquelle R⁵ est identique à celui décrit ci-dessus, et
le composé d'acide carboxylique représenté par la formule (7) est un composé d'acide carboxylique représenté par la formule (11) : 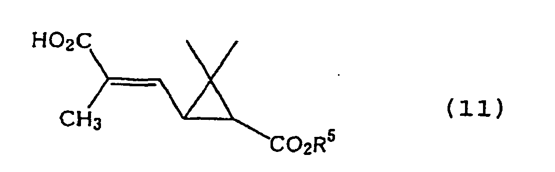 dans laquelle R⁵ est identique à celui décrit ci-dessus.

15. Procédé selon la revendication 13, dans lequel
la quantité du composé d'hydroxyperoxyde organique à utiliser est de 2 à 3,5 mol par rapport à une mole du composé d'oléfine représenté par la formule (4), et
un constituant principal est le composé d'aldéhyde représenté par la formule (6).

16. Procédé selon la revendication 14, dans lequel
la quantité du composé d'hydroxyperoxyde organique à utiliser est de 2 à 3,5 mol par rapport à une mole du composé d'acide chrysanthémique représenté par la formule (8), et
un constituant principal est le composé d'aldéhyde représenté par la formule (10).

17. Procédé de production d'un composé contenant de l'oxygène comprenant la réaction d'un composé organique avec un agent oxydant en présence d'une composition de catalyseur d'oxydation comprenant un mélange d'un composé de sélénium, d'un composé aromatique contenant de l'azote et d'un acide, dans lequel
le composé de sélénium est le dioxyde de sélénium ou un séléniure de métal alcalin,
le composé aromatique contenant de l'azote est la pyridine, la 2-méthylpyridine, la 3-butylpyridine, la collidine, la quinoléine, la 1,10-phénanthroline, l'imidazole, le N-méthylimidazole, le 2-éthyl-4-méthylimidazole ou le benzimidazole,
l'acide est l'acide tétrafluoroborique, l'acide phosphorique ou l'acide benzènesulfonique,
le composé organique est un composé de cycloalcanone,
l'agent oxydant est le peroxyde d'hydrogène, et
le composé contenant de l'oxygène est un acide cycloalcanecarboxylique.

18. Procédé selon la revendication 17, dans lequel
le composé de cycloalcanone est un composé de cycloalcanone représenté par la formule (12) : 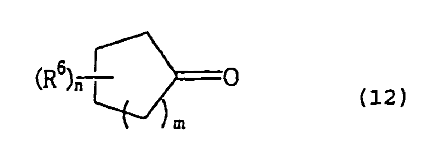 dans laquelle R⁶ représente un atome d'hydrogène ; un atome d'halogène ; un groupe alkyle en C1-C20 ; un groupe aryle en C6-C10, un groupe aralkyle en C7-C12 et un groupe alcoxy en C1-C20 et les groupe alkyle en C1-C20, groupe aryle en C6-C10, groupe aralkyle en C7-C12 et groupe alcoxy en C1-C20 cités ci-dessus peuvent ici être substitués avec un atome ou des atomes d'halogène, ou un groupe ou des groupes alcoxy en C1-C20, m représente un nombre entier de 0 à 8, n représente un nombre entier de 0 à 11 qui satisfait n ≤ m+3, et lorsque n est égal à 2 ou plus, les R⁶ peuvent ici être identiques ou différents, deux R⁶ quelconques peuvent être liés pour former un noyau, et
le composé d'acide cycloalcanecarboxylique est un compsoé d'acide cycloalcanecarboxylique représenté par la formule (13) : 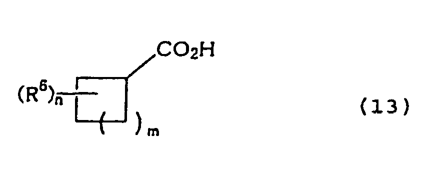 dans laquelle R⁶, m et n sont identiques à ceux décrits ci-dessus.
